Europäisches Patentamt

European Patent Office

Office européen des brevets

(11) Publication number: **0 443 560 A2**

# EUROPEAN PATENT APPLICATION

(21) Application number: 91102497.4

(22) Date of filing: 21.02.91

(51) Int. Cl.5: **C07K 5/02**, A61K 37/02, C07K 1/00

(30) Priority: 23.02.90 US 484016

(43) Date of publication of application:
28.08.91 Bulletin 91/35

(84) Designated Contracting States:
AT BE CH DE DK ES FR GB GR IT LI LU NL SE

(71) Applicant: BIO-MEGA INC.
2100 Rue Cunard
Laval Québec H7S 2G5(CA)

(72) Inventor: Anderson, Paul Cates
4130 Edward Higgins
Pierrefonds, Quebec, H8Y 3M8(CA)
Inventor: Rakhit, Sumanas
1277 Tecumseh
Dollard des Ormeaux, Quebec, H9A 3A5(CA)
Inventor: Yoakim, Christiane
63 Gianchetti
Laval, Quebec, H7G 4T5(CA)

(74) Representative: Laudien, Dieter, Dr. et al
Boehringer Ingelheim GmbH, Abteilung
Patente
W-6507 Ingelheim am Rhein(DE)

(54) HIV protease inhibitors.

(57) Disclosed herein are compounds of the general formula X-A-B-Y wherein X is $R^1OC(O)$, $R^1NHC(O)$ or $R^1C(O)$ wherein $R^1$ is, for example, alkyl, optionally substituted phenyl or optionally substituted benzyl, A is a non-peptide linking unit, e.g. statyl or Phe$\Psi$[CH$_2$NH]Leu, B is an amino acid residue of glutamic acid or a related derivative thereof, and Y is alkoxy, cycloalkoxy, a substituted alkoxy or a substituted amino group, e.g. cycloalkylalkoxy, aralkoxy, alkylamino, (cycloalkyl)alkylamino or aralkylamino. The compounds inhibit the activity of human immunodeficiency virus (HIV) protease and interfere with HIV induced cytopathogenic effects in human cells. These properties render the compounds used for combating HIV infections.

EP 0 443 560 A2

Field of the Invention

This invention relates to compounds exhibiting activity against particular retroviruses, to process for producing the compounds, to pharmaceutical preparations thereof, and to the use of the compounds for the manufacture of a medicament for combating infections caused by the retroviruses.

Background of the Invention

During the last ten years, increasing attention has been focused on retroviruses. These viruses cause a variety of diseases in vertebrates, the most insidious to humans being immunodeficiencies and cancers. In 1983, a retrovirus, known as human immunodeficiency virus type 1 (HIV- 1), was established as a causative agent for acquired immune deficiency syndrome (AIDS). This virus has become a pestilence of alarming proportion. More recently, the closely related virus, human immunodeficiency virus type 2 (HIV-2) has been identified as a second causitive agent of AIDS. (Hereinafter, the term "HIV" is meant to include both HIV-1 and HIV-2 and any mutants thereof.)

Presently, several compounds are being evaluated in the clinic as possible therapeutic agents for AIDS. Another compound, 3-deoxythymidine (known also as zidovudine or AZT), has been shown in the clinic to decrease mortality and the frequency of opportunistic infections in AIDS patients. This latter compound is being used to manage certain patients with symptomatic HIV infections. However, in spite of some recent progress, the need for an effective therapy for AIDS still exists. For recent reviews, see R.A. Weiss in "Molecular Basis of Virus Disease", Symposium of the Society for General Microbiology, Vol. 40, Eds. W.C. Russel and J.W. Almond, University Press, Cambridge, UK, 1987, pp 167-192, and R.C. Gallo and L. Montagnier, Scientific American, 259, (4), 40 (1988).

One approach to finding agents having anti-HIV activity is to inhibit the action of HIV-encoded enzymes. This manner of inhibition interferes with the replication and propagation of the virus. Such an approach has been applied successfully in a search for inhibitors of the viral encoded enzyme, reverse transcriptase (RT). More explicitly, the previously noted zidovudine was found to inhibit RT which is required for viral replication. Subsequently, zidovudine was developed as an anti-HIV agent. Still more recently, this approach has been investigated using another HIV-encoded enzyme known as HIV protease as the target enzyme. In one instance, pepstatin A was found to inhibit the intracellular processing of the HIV gag proteins. See, S. Seelmeier et al., Proc. Natl. Acad. Sci. USA, 85, 6612 (1988). However, the development of pepstatin A as an anti-HIV agent seems improbable in view of its multiple activities. Subsequently, M.L. Moore et al., Biochem. Biophys. Res. Comm., 159, 420 (1989) and G.B. Dreyer et al., Proc. Natl. Acad. Sci. USA, 86, 9752 (1989) reported on investigations showing the inhibition of HIV protease by hexapeptide and heptapeptide analogs. A.D. Richards et al., FEBS Letters 247, 113 (1989), also have reported that acetyl-pepstatin and an octapeptide analog inhibit HIV protease in vitro. Similarly, in vitro inhibition of HIV protease activity by modified peptide substrates, based on amino acid sequences of six to ten amino acids of the HIV polyprotein, has been reported by S. Billich et al., J. Biol. Chem., 263, 17905 (1988) and by M. Kotler et al., Proc. Natl. Acad. Sci. USA 85, 4185 (1988). The recent publication of patent applications, typically B. Weidmann, UK patent application 2,203,740, published October 26, 1988, I.S. Sigal et al., European patent application 337,714, published October 18, 1989 and D.J. Kempf et al., PCT patent application WO 89/10752, published November 16, 1989, disclosing peptide-like compounds with retroviral protease inhibition, is indicative of the increasing interest in this area.

The present application discloses a group of compounds which are potent inhibitors of HIV protease. Moreover, a capacity to inhibit HIV induced cytopathogenic effects in human cells has been demonstrated for many of the compounds. Such properties, together with the attributes of a relatively selective action and an apparent lack of toxicity, renders the compounds useful as agents for combating HIV infections.

The compounds of this invention can be considered to have an incidental relationship to peptide derivatives since they have an amino acid residue and/or derived amino residues incorporated into their structure and as such have a partial structural resemblance to pepstatin A and the previously noted peptide analogs. The present compounds also possess a partial structural resemblance to peptide derivatives reported to be renin inhibitors; for instance, see D.F. Veber et al., European patent application 77,028, published April 20, 1983, R.E. Ten Brink, PCT patent application WO 87/02986, published May 21, 1987, P. Raddatz, Australian patent application 73833/87, published December 17, 1987, J.P. Hudspeth et al., US patent 4,743,585, issued May 10, 1988, W.J. Greenlee et al., European patent application 273,696, published July 6, 1988, and A. Wagner et al., Australian patent application 76241/87, published February 4, 1988. The remaining structural features and differences in biological profiles distinguish the prior art compounds from the present peptide derivatives, notwithstanding the existence of broad generic disclosures such as found in the previously noted PCT patent application of Ten Brink and the European patent application of Sigal et al., encompassing an almost infinite number of compounds.

## Summary of the Invention

The compounds of this invention are represented by formula 1

X-A-B-Y     (1)

wherein X is $R^1OC(O)$, $R^1NHC(O)$ or $R^1C(O)$ wherein $R^1$ is

(i) lower alkyl,

(ii) lower cycloalkyl,

(iii) (lower cycloalkyl)-(lower alkyl),

(iv) phenyl or phenyl monosubstituted with hydroxy, lower alkyl, lower alkoxy or halo, or

(v) phenyl(lower)alkyl or phenyl(lower)alkyl monosubstituted on the aromatic portion thereof with hydroxy, lower alkyl, lower alkoxy or halo;

A is a derived amino acid radical of the formula $NHCH(R^2)$-T wherein $R^2$ has the same meaning as defined hereinabove for $R^1$ and T is $CH(OH)CH_2C(O)$, $CH(OH)CH_2CH_2C(O)$ or $CH_2NHCH(R^3)C(O)$ wherein $R^3$ has the same meaning as defined hereinabove for $R^1$, or A is a derived amino acid radical of the formula $NHCH[CH(OH)-R^{3A}]CH(OH)CH_2(O)$ wherein $R^{3A}$ has the same meaning as defined hereinabove for $R^1$, the three asymmetric centers of the tertiary carbon atoms depicted for the last said radical having in order the (S), (R) and (S) chiral configurations;

B is $NHCH(R^4)C(U)$ wherein $R^4$ is $CR^5(R^6)CR^7(R^8)C(O)V$ wherein $R^5$, $R^6$, $R^7$ and $R^8$ each independently is hydrogen or lower alkyl and V is hydroxy or lower alkoxy, and U is oxo or thioxo; and

Y is $OR^9$ wherein $R^9$ is

(i) (1-10C)alkyl,

(ii) lower cycloalkyl,

(iii) (lower cycloalkyl)-(lower alkyl),

(iv) phenyl(lower)alkyl or phenyl(lower)alkyl wherein the aromatic portion thereof is monosubstituted with hydroxy, halo, lower alkoxy, lower alkyl, $SO_2NH_2$ or $S(O)_nR^{10}$ wherein n is 0, 1 or 2 and $R^{10}$ is lower alkyl,

(v) (Het)-lower alkyl wherein Het represents a five or six membered heterocyclic radical containing one or two heteroatoms selected from nitrogen, oxygen and sulfur, or

(vi) $CH_2CH_2O-(CH_2)_m$-Ph wherein m is 0 or 1, or $CH_2CH_2O-(CH_2)_m$-Ph wherein the aromatic portion (Ph) thereof is monosubstituted with hydroxy, halo, lower alkoxy or lower alkyl and m is 0 or 1; or

Y is $NR^{11}R^{12}$ wherein $R^{11}$ is hydrogen or lower alkyl and $R^{12}$ has the same meaning as defined hereinabove for $R^9$; or a therapeutically acceptable salt thereof.

A preferred group of the compounds of this invention is represented by formula 1 wherein X is $R^1OC(O)$, $R^1NHC(O)$ or $R^1C(O)$ wherein $R^1$ is

(i) lower alkyl,

(ii) cyclopropyl, cyclopentyl or cyclohexyl,

(iii) cyclopropylmethyl, cyclohexylmethyl or 2-cyclohexylethyl,

(iv) phenyl, 4-chlorophenyl, 4-methylphenyl or 4-methoxy-phenyl, or

(v) benzyl, (4-chlorophenyl)methyl, (4-methylphenyl)methyl or (4-methoxyphenyl)methyl;

A is $NHCH(R^2)$-T wherein $R^2$ lower alkyl, (lower cyclealkyl)methyl, 2-(lower cycloalkyl)ethyl, benzyl, (4-hydroxyphenyl)methyl, (4-fluorophenyl)methyl, or (4-methoxyphenyl)methyl and T is $CH(OH)CH_2C(O)$, $CH(OH)CH_2CH_2C(O)$ or $CH_2NHCH(R^3)C(O)$ wherein $R^3$ is lower alkyl, cyclopropylmethyl, cyclohexylmethyl, phenyl or benzyl, or A is (S,R,S)-$NHCH[CH(OH)-(lower cycloalkyl)]CH(OH)CH_2C(O)$;

B is $NHCH(R^4)C(U)$ wherein $R^4$ is $CH_2CH_2C(O)OH$, $CH_2CH(CH_3)C(O)OH$, $CH(C_2H_5)CH_2C(O)OH$, $CH_2CH_2C(O)OCH_3$, $CH_2CH_2C(O)OC_2H_5$, $CH_2CH(CH_3)C(O)OCH_3$, or $CH(CH_3)CH(CH_3)C(O)OCH_3$, and U is oxo or thioxo; and

Y is $OR^9$ wherein $R^9$ is

(i) (1-10C)alkyl,

(ii) cyclopropyl, cyclopentyl or cyclohexyl,

(iii) cyclopropylmethyl, 2-cyclopropylethyl, 3-cyclopropylpropyl, cyclohexylmethyl, 2-cyclohexylethyl or 3-cyclohexylpropyl,

(iv) phenyl(lower)alkyl or phenyl(lower)alkyl substituted at position 4 of the phenyl with hydroxy, fluoro, chloro, bromo, lower alkoxy, lower alkyl, $SO_2NH_2$, lower alkylthio or lower alkylsulfonyl,

(v) Het-lower alkyl wherein Het is a heterocyclic radical selected from 2-pyrrolyl, 2-furyl, 2-thienyl, 2-isoxazolyl and 2-thiazolyl, or

(vi) $CH_2CH_2O(CH_2)_m$-Ph wherein m is 0 or 1 or $CH_2CH_2O(CH_2)_m$-Ph wherein the aromatic portion thereof is monosubstituted with hydroxy, fluoro, chloro, bromo, lower alkoxy or lower alkyl; or

Y is $NR^{11}R^{12}$ wherein $R^{11}$ is hydrogen or methyl and $R^{12}$ has the same meaning as defined in the last instance for $R^9$;

or a therapeutically acceptable salt thereof.

A more preferred group of the compounds is represented by formula 1 wherein X is $R^1OC(O)$ or $R^1C(O)$ wherein $R^1$ is methyl, ethyl, 1-methylethyl, 1,1-dimethylethyl, propyl, 1-methylpropyl, 1-ethylpropyl, 2-methylpropyl, 1,1-dimethylpropyl, 1,2-dimethylpropyl, 2,2-dimethylpropyl, butyl, cyclopropyl, cyclohexyl, cyclopropylmethyl, cyclohexylmethyl, phenyl, 4-methoxyphenyl, benzyl, (4-chlorophenyl)methyl or (4-methoxyphenyl)methyl; A is $NHCH(R^2)$-T wherein $R^2$ is 1-methylethyl, propyl, 1-methylpropyl, 2-methylpropyl, 1,1-dimethylethyl, 2,2-dimethylpropyl, butyl, cyclopropylmethyl, cyclohexylmethyl, 2-cyclohexylethyl, benzyl, (4-hydroxyphenyl)methyl, (4-fluorophenyl)methyl or (4-methoxyphenyl)methyl, and T is $CH(OH)$-$CH_2C(O)$, $CH(OH)CH_2CH_2C(O)$ or $CH_2NHCH(R^3)C(O)$ wherein $R^3$ is 1-methylethyl, propyl, 1-methylpropyl, 2-methylpropyl, 1,1-dimethylethyl, butyl, cyclopropylmethyl, cyclohexylmethyl, phenyl or benzyl, or A is (S,R,S)-NHCH[CH(OH)-cyclohexyl]CH(OH)CH$_2$C(O); B is $NHCH(R^4)C(U)$ wherein $R^4$ is $CH_2CH_2C(O)OH$, $CH_2CH_2C(O)OMe$, $CH_2CH(CH_3)C(-O)OH$ or $CH_2CH(CH_3)C(O)OCH_3$ and U is oxo; and Y is methoxy, ethoxy, hexyloxy, octyloxy, cyclopropyloxy, 3-cyclopropylpropoxy, 2-cyclohexylethoxy, 3-cyclohexylpropoxy, 2-phenylethoxy, 2-[4-(aminosulfonyl)phenyl]ethoxy, 2-(4-fluorophenyl)ethoxy, 2-[4-(methylthio)phenyl]ethoxy, 3-phenylpropoxy, 3-(4-fluorophenyl)propoxy, 3-(4-chlorophenyl)propoxy, 3-(4-hydroxyphenyl)propoxy, 3-(4-propoxyphenyl)propoxy, 3-[4-(methylthio)phenyl]propoxy, 3-[4-(methylsulfonyl)phenyl]propoxy,2-(phenoxy)-ethoxy, 2-(2-chlorophenoxy)ethoxy, 2-(4-methylphenoxy)ethoxy, 2-(phenylmethoxy)ethoxy, butylamino, hexylamino, octylamino, cyclopropylamino, 3-cyclopropylpropylamino, 2-cyclohexylethylamino, 3-cyclohexylpropylamino, 2-phenylethylamino, 2-[4-(aminosulfonyl)-phenyl]ethylamino, 2-(4-fluorophenyl)ethylamino, 2-(4-chlorophenyl)ethylamino, 2-[4-(methylthio)phenyl]ethylamino, 2-(4-methoxyphenyl)ethylamino, 3-phenylpropylamino, 3-(4-fluorophenyl)propylamino, 3-(4-chlorophenyl)propylamino, 3-(4-hydroxyphenyl)-propylamino, 3-(4-methoxyphenyl)propylamino, 3-(4-propoxyphenyl)propylamino, 3-[4(methylthio)phenyl]-propylamino, 3-[4-(methylsulfonyl)phenyl]propylamino, 3-(2-furyl)propylamino, 3-(2-thienyl)propylamino, 2-(phenoxy)-ethylamino, 2-(2-chlorophenoxy)ethylamino, 2-(4-methylphenoxy)ethylamino or 2-phenylmethoxy)-ethylamino; or a therapeutically acceptable salt thereof.

A most preferred group of the compounds is represented by formula 1 wherein X is methoxycarbonyl, 1-methylethoxycarbonyl, 1,1-dimethylethoxycarbonyl, 1-oxobutyl, 3-methyl-1-oxobutyl, 2-ethyl-1-oxobutyl, 2,2-dimethyl-1-oxobutyl, 2,3-dimethyl-1-oxobutyl, 3,3-dimethyl-1-oxobutyl or cyclohexylcarbonyl; A is the radical $NHCH(R^2)CH(OH)CH_2C(O)$ wherein $R^2$ is 2,2-dimethylpropyl, butyl, cyclopropylmethyl, cyclohexylmethyl or 2-cyclohexylethyl, the two asymmetric centers of the last said radical having the (S) chiral configurations, or A is the radical $NHCH(R^2)CH_2$-$NHCH(R^3)C(O)$ wherein $R^2$ is cyclohexylmethyl, benzyl or (4-fluorophenyl)methyl and $R^3$ is 1-methylethyl, 2-methylpropyl, butyl or cyclopropylmethyl, the two asymmetric centers of the last said radical having the (S) chiral configuration, or A is (S,R,S)-NHCH[CH(OH)-cyclohexyl]-CH(OH)CH$_2$C(O); B is Glu, Glu(OMe) or NHCH[CH$_2$CH(CH$_3$)-COOH]C(O);and Y is 2-phenylethoxy, 3-phenylpropoxy, hexylamino, octylamino, 3-cyclohexylpropylamino, 2-phenylethylamino, 2-(4-fluoro-phenyl)ethylamino, 2-(4-chlorophenyl)ethylamino, 2-[4-(methylthio)phenyl]ethylamino, 2-(4-methoxyphenyl)ethylamino, 3-phenylpropylamino, 3-(4-fluorophenyl)propylamino, 3-(4-hydroxyphenyl)propylamino, 3-(4-propoxyphenyl)propylamino, 3- [4-(methylthio)phenyl]propylamino, 3-(2-furyl)propylamino, 3-(2-thienyl)-propylamino, 2-(phenoxy)ethylamino, 2-(2-chlorophenoxy)-ethylamino or 2-(phenylmethoxy)ethylamino; or a therapeutically acceptable salt thereof.

Included within the scope of this invention is a pharmaceutical composition for treating HIV infections in a human comprising a compound of formula 1, or a therapeutically acceptable salt thereof, and a pharmaceutically acceptable carrier.

The scope of the invention includes as well the use of said compounds for the manufacture of a medicament for treating HIV infections in a human, comprising an effective amount of the compound of formula 1, or a therapeutically acceptable salt thereof.

Also included within the scope is the use of said compounds for the manufacture of a medicament for protecting human cells against HIV pathogenesis, comprising an anti-HIV effective amount of a compound of formula 1, or a therapeutically acceptable salt thereof.

Processes for preparing the compounds of formula 1 are described hereinafter.

Details of the Invention

GENERAL

The term "residue" with reference to an amino acid means a radical derived from the corresponding α-amino acid by eliminating the hydroxyl of the carboxy group and one hydrogen of the α-amino group.

In general, the abbreviations used herein for designating the amino acids and the protective groups are based on recommendations of the IUPAC-IUB Commission of Biochemical Nomenclature, see European Journal of Biochemistry, 138, 9 (1984). For instance, Val, Glu, Tyr, Ala, Ile, Asp, Phe, Leu and Gly represent the residues of L-valine, L-glutamic acid, L-tyrosine, L-alanine, L-isoleucine, L-aspartic acid, L-phenylalanine, L-leucine and glycine, respectively. The symbols "Cpa" and "Cha" represent the residues of 2(S)-amino-3-cyclopropylpropanoic acid (L-cyclopropylalanine) and 2(S)-amino-3-cyclohexylpropanoic acid (L-cyclohexylalanine), respectively. The symbols "Nle", "Nva" and "Tbg" represent the residues of 2(S)-aminohexanoic acid (L-norleucine), 2(S)-aminopentanoic acid (L-norvaline) and 2(S)-amino-3,3-dimethylbutanoic acid, respectively. The symbol "Tyr(Me)" represents the residue of $O^4$-methyltyrosine. The symbol "Glu(OMe)" represents the residue of $O^5$-methyl hydrogen glutamate. The symbol "4F-Phe" represents the residue of 2-(S)-amino-3-(4-fluorophenyl)propanoic acid.

The symbol "Ψ[CSNH]" used between the three letter representations of two amino acid residues means that the normal amide bond between those residues has been replaced with a thioamide bond.

The term "lower alkyl" as used herein, either alone or in combination with a radical, means straight chain alkyl radicals containing one to six carbon atoms and branched chain alkyl radicals containing three to four carbon atoms and includes methyl, ethyl, propyl, butyl, hexyl, 1-methylethyl, 1-methylpropyl, 2-methylpropyl and 1,1-dimethylethyl.

The term "(1-10C)alkyl" as used herein means a straight chain or branched chain alkyl radical containing one to ten carbon atoms and includes, for example, methyl, ethyl, hexyl, octyl, decyl, 1-methylpropyl and 4-methylpentyl.

The term "lower cycloalkyl" as used herein, either alone or in combination with a radical, means saturated cyclic hydrocarbon radicals containing from three to six carbon atoms and includes cyclopropyl, cyclobutyl, cyclopentyl and cyclohexyl.

The term "lower alkoxy" as used herein means straight chain alkoxy radicals containing one to six carbon atoms and branched chain alkoxy radicals containing three to four carbon atoms and includes methoxy, ethoxy, propoxy, hexoxy, 1-methylethoxy, butoxy and 1,1-dimethylethoxy. The latter radical is known commonly as tertiary-butyloxy.

The term "halo" as used herein means a halo radical selected from bromo, chloro, fluoro or iodo.

The symbol "Boc" represents 1,1-dimethylethoxycarbonyl, known commonly as tertiary-butyloxycarbonyl. The symbol "Moc" represents methoxycarbonyl. The symbol "Ipoc" represents 1-methylethoxycarbonyl. The symbol "Ph" represents a phenyl radical. Other symbols include "2Cl-Ph" for 2-chlorophenyl, "4F-Ph" for 4-fluorophenyl, "4HO-Ph" for 4-hydroxyphenyl,"4MeS-Ph" for 4-(methylthio)phenyl, "4PrO-Ph" for 4-propoxyphenyl and "4NH₂SO₂-Ph" for 4-(aminosulfonyl)phenyl.

With reference to the symbol A of general formula 1, the radical "-NHCH($R^2$)-T-" wherein $R^2$ is as defined hereinabove and T is $CH(OH)CH_2C(O)$ represents the radical derived from the amino acid known as statine (i.e. 4(S)-amino-3(S)-hydroxy-6-methylheptanoic acid) and its close analogs. The radical is derived by eliminating the hydroxyl of the carboxy group and one hydrogen of the amino group of the corresponding γ-amino acid. Each such radical has two chiral centers and thus can exist in various optically active or optically inactive forms. All forms are included for the compounds of formula 1 and for the appropriate intermediates therefor, the 4(S)-amino-3(S)-hydroxy enantiomers being preferred. The requisite 4-amino-3-hydroxy pentanoic acids for preparing the synthon to incorporate the radical into the compound of formula 1 can be prepared by methods described by D.H. Rich and E.T.O. Sun, J. Med. Chem., 23, 27 (1980), and references therein; see also A.H. Fray et al., J. Org. Chem., 51, 4828 (1986), and references therein.

The term "Sta" represents the radical -NHCH(2-methylpropyl)CH(OH)CH₂C(O)-, derived from statine. The term "Nor- Sta" represents the radical -NHCH(CH₂CH₂CH₂CH₃) CH(OH)CH₂C(O)-, derived from 4-amino-3-hydroxyoctanoic acid. The term "ACPPA" represents the radical -NHCH(cyclopropylmethyl)CH-(OH)CH₂C(O)-,derived from 4-amino-5-cyclopropyl-3-hydroxypentanoic acid. The term "ACHPA" represents the radical -NHCH(cyclohexylmethyl)-CH(OH)CH₂C(O)-, derived from 4-amino-5-cyclohexyl-3-hydroxypentanoic acid. The term "homoACHPA" represents the radical -NHCH(CH₂CH₂-cyclohexyl)CH(OH)CH₂C(O), derived from 4-amino-6-cyclohexyl-3-hydroxyhexanoic acid. The term "AHPPA" represents the radical -NHCH(benzyl)CH(OH)CH₂C(O)-, derived from 4-amino-3-hydroxy-5-phenylpentanoic acid and the term "AHDHA" represents the radical -NH-CH-[CH₂C-(CH₃)₃]CH-(OH)CH₂CO-, derived from 4-amino-3-hydroxy-6,6-dimethylheptanoic acid. The 4(S)-amino-3(S)-hydroxy enantiomers of these last seven embodiments are preferred. Unless designated otherwise by an antecedent such as (3R, 4R), the terms Sta, NorSta, ACPPA, ACHPA, homoACHPA, AHPPA and AHDHA represent their respective 4(S)-amino-3(S)-hydroxy enantiomers. The term "HACHPA" represents the radical -NHCH[CH(OH)-cyclohexyl]CH(OH)CH$_2$CO-, derived from (3S,4R,5S)-amino-5-cyclohexyl-3,5-dihydroxypentanoic acid.

Also note that when A is the radical "NHCH(R$^2$)-T" wherein R$^2$ is as defined hereinabove and T is CH$_2$NHCH(R$^3$)C(O) wherein R$^3$ is as defined hereinabove, the radical is equivalent to two adjoining, corresponding amino acid residues (or derived amino acids residues) wherein the amide bond joining the two residues is reduced. According to convention, the latter radical can be expressed symbolically as two amino acid residues (in the three letter system) with the symbol "Ψ[CH$_2$NH]" inserted between the designation of the two adjoining amino acid residues. In this instance, the radical can be derived from α-amino acids having the (R)- or preferably the (S)-chiral configuration for the α-carbon atom. Accordingly, for example, the peptide of formula 1 wherein X is R$^1$OC(O) wherein R$^1$ is 1,1-dimethylethyl, A is NHCH-(benzyl)CH$_2$NHCH(2-methylpropyl)C(O) with two (S)-asymmetric centers, B is Glu and Y is 3-phenylpropylamino can be designated as Boc-PheΨ[CH$_2$NH]Leu-Glu-NHCH$_2$CH$_2$CH$_2$Ph.

Similarly, according to convention, when A is the radical "NHCH(R$^2$)CH(OH)CH$_2$CH$_2$C(O)" wherein R$^2$ is as defined hereinabove, the radical can be considered as an amino acid or derived amino acid residue joined to a glycine residue wherein the amide bond normally joining the two residues is replaced by "CH(OH)CH$_2$". Hence, the latter radical can be expressed as two amino acid residues in the three letter system with the symbol "Ψ[CH(OH)CH$_2$]" inserted between the two amino acid symbols. For example, the peptide of formula 1 where X is R$^1$OC(O) wherein R$^1$ is 1,1-dimethylethyl, A is NHCH(CH$_2$-cyclohexyl)-CH(OH)-CH$_2$CH$_2$C(O) with two (S)-asymmetric centers, B is Glu and Y is 3-phenylpropylamino can be designated as Boc-ChaΨ[CH(OH)CH$_2$]Gly-Glu-NHCH$_2$CH$_2$CH$_2$Ph.

With reference to the compounds of formula 1, note that the asymmetric α-carbon atoms of the amino acid residues or derived amino acid residues represented by the symbol B have the S configuration, whereas asymmetric carbon atoms, residing in the side chain of these amino acid or derived amino acid residues also may have the R configuration.

The term "coupling agent" as used herein means an agent capable of effecting the dehydrative coupling of a free carboxy group of one reactant with a free amino group of another reactant to form an amide bond between the reactants. The agents promote or facilitate the dehydrative coupling by activating the carboxy group. Descriptions of such coupling agents and activated groups are included in general textbooks of peptide chemistry; for instance, E. Schroder and K.L. Lubke, "The Peptides", Vol. 1, Academic Press, New York, N.Y., 1965, pp 2-128, and K.D. Kopple, "Peptides and Amino acids", W.A. Benjamin, Inc., New York, N.Y., 1966, pp 33-51. Examples of coupling agents are thionyl chloride, diphenylphosphoryl azide, dicyclohexylcarbodiimide, N-hydroxysuccinimide, or 1-hydroxybenzotriazole in the presence of dicyclohexylcarbodiimide. A very practical and useful coupling agent is (benzotriazol-1-yloxy)tris-(dimethylamino)phosphonium hexafluorophosphate, described by B. Castro et al., Tetrahedron Letters, 1219 (1975), see also D. Hudson, J. Org. Chem., 53, 617 (1988), either by itself or in the presence of 1-hydroxybenzotriazole.

The term "pharmaceutically acceptable carrier" as used herein means a non-toxic, generally inert vehicle for the active ingredient, which does not adversely affect the ingredient.

The term "effective amount" as used herein means a predetermined amount of the compound of this invention sufficient to be effective against HIV in vivo.

PROCESS

The compounds of formula 1 can be prepared by processes which incorporate therein methods commonly used in peptide synthesis such as the classical solution coupling of amino acid residues and/or peptide fragments. Such methods are described, for example, by E. Schroder and K. Lubke, cited above, in the textbook series, "The Peptides: Analysis, Synthesis, Biology", E. Gross et al., Eds., Academic Press, New York, N.Y., 1979-1987, Volumes 1 to 8, and by J.M. Stewart and J.D. Young in "Solid Phase Peptide Synthesis", 2nd ed., Pierce Chem. Co., Rockford, IL, USA, 1984.

A common feature of the aforementioned processes for preparing the present compounds is the protection of any labile side chain groups of the reactants with suitable protective groups which will prevent a chemical reaction from occurring at that site until the protective group is ultimately removed. Usually also common is the protection of an α-amino group on an amino acid or a fragment while that entity reacts at the carboxy group, followed by the selective removal of the α-amino protective group to allow subsequent reaction removal of the α-amino protective group to allow subsequent reaction to take place at that location.

More explicitly, a convenient and practical preparation of the compounds of formula 1 involves a key step which uses a coupling agent to couple a first fragment of formula X-A-OH wherein X and A are as defined herein with a second fragment of formula H-B$^1$-Y wherein B$^1$ has the same meaning as defined hereinabove for B except that the side chain carboxy or ester (alkoxycarbonyl) group is replaced by a protected carboxyl and Y is as defined herein. The coupling of these two fragments provides the corresponding protected intermediate of formula X-A-B$^1$-Y wherein X, A, B$^1$ and Y are as defined herein. Thereafter, the protected intermediate is subjected to carboxy deprotection, and to esterification of the carboxy group and/or to resolution if required, to give the corresponding compound of formula 1.

Still more explicitly with regard to the characterization of the preceding second fragment, B$^1$ represents the radical NHCH(R$^{4A}$)C(U) wherein R$^{4A}$ is CR$^5$(R$^6$)CR$^7$(R$^8$)C(O)V$^1$ wherein R$^5$ to R$^8$, inclusive, are as defined hereinabove and V$^1$ is a carboxy protective group, for example, benzyloxy, cyclohexyloxy or 2,6-dichlorobenzyloxy, and U is oxo or thioxo. Accordingly, the second fragment can, depending on whether U is oxo or thio, contain an ester bond, a thioester bond, an amide bond or a thioamide bond, respectively.

The aforementioned first fragment of formula X-A-OH is prepared by either of two conventional processes depending on whether A is the radical NHCH(R$^2$)CH(OH)CH$_2$C(O) or NHCH(R$^2$)CH$_2$NHCH(R)$^3$C-(O). In the first case, i.e. A is NHCH(R$^2$)CH(OH)CH$_2$C(O), the first fragment can be obtained by the reaction of the appropriate 4-amino-3-hydroxypentanoic acid (having carboxy or hydroxy protection if desired or required) with an appropriate reactant for forming the urethane, urea or amide bond between the two reactants, followed by hydroxy and/or carboxy deprotection, if required. Such reactions are well known in the art; for example, the reaction di-t-butyl dicarbonate with 4-amino-5-cyclohexyl-3-hydroxypentanoic acid in the presence of an organic base to give Boc-ACHPA-OH. In the second case, i.e. A is NHCH(R)-$^2$CH$_2$NHCH(R$^3$)C(O), the first fragment can be obtained by the reductive N-alkylation between two reactants; one reactant bearing the terminal urethane, urea or amide portion for the final product, and each reactant containing a precursor portion of the A unit whereby the CH$_2$NH bond of the A unit is formed. For example, the reductive N-alkylation of the p-nitrobenzyl ester of Leu-OH with Boc-phenylalaninal in the presence of sodium cyanoborohydride, followed by deprotection of the C-terminal carboxy of the intermediate, gives the corresponding first fragment, i.e. Boc-PheΨ[CH$_2$NH]Leu-OH (see example 1B and 1C).

Likewise, the aforementioned second fragment of formula H-B$^1$-Y is prepared by conventional processes. A convenient process for the preparation of second fragments in which Y is a substituted amino involves the formation of an amide bond between two reactants representing precursor portions for the amino acid unit B and the amine unit Y, followed if required by the conversion of the amide bond to a thioamide bond. More specifically, an amino acid derivative of the formula apg-NH-CH(R$^{4A}$)C(O)OH in which apg is an α-amino protective group (e.g. Boc) and R$^{4A}$ is as defined herein is coupled, by means of a coupling agent, with the appropriate amine of formula HNR$^{11}$R$^{12}$ wherein R$^{11}$ and R$^{12}$ are as defined herein to give the corresponding protected amide of formula apg-NHCH(R$^{4A}$)C(O)NR$^{11}$R$^{12}$, which on amino deprotection yields the corresponding second fragment containing the amide bond. When the corresponding second fragment containing a thioamide bond is desired, the last-named protected amide is reacted with an agent capable of converting an amide group to a thioamide group, e.g. phosphorus pentasulfide or preferably Lawesson's reagent [see U. Pederson et al., Tetrahedron, 38, 3267 (1982)], to obtain the corresponding protected thioamide of formula apg-NHCH(R$^{4A}$)C(S)NR$^{11}$R$^{12}$ which on amino deprotection gives the corresponding second fragment containing a thioamide bond.

For the preparation of second fragments of formula H-B$^1$-Y in which Y is lower alkoxy, lower cycloalkoxy, etc,. the same processes as those described for the second fragment (Y = substituted amino) can be used except that the appropriate alcohol of formula R$^9$OH replaces the amine of formula HNR$^{11}$R$^{12}$. In this manner, the corresponding second fragments containing an ester or thioester bond are obtained.

Thereafter, the above described first and second fragments are utilized for the key coupling step, described above, to provide the compounds of formula 1.

The compound of formula 1 of this invention can be obtained in the form of a therapeutically acceptable salt. In the instance where a particular peptide has a residue which functions as a base, examples of such salts are those with organic acids, e.g. acetic, lactic, succinic, benzoic, salicylic, methanesulfonic or p-toluenesulfonic acid, as well as polymeric acids such as tannic acid or carboxymethyl cellulose, and also salts with inorganic acids such as hydrohalic acids, e.g. hydrochloric acid, or sulfuric acid, or phosphoric acid. If desired, a particular acid addition salt is converted into another acid addition salt, such as a non-toxic, pharmaceutically acceptable salt, by treatment with the appropriate ion exchange resin in the manner described by R.A. Boissonnas et al., Helv. Chim. Acta, 43, 1849 (1960). In the instance where a particular peptide has one or more free carboxy groups, examples of such salts are those with the sodium, potassium or calcium cations, or with organic bases, for example, triethylamine or N-methylmorpholine.

In general, the therapeutically acceptable salts of the peptides of formula 1 are biologically fully equivalent to the peptides themselves.

## BIOLOGICAL ASPECTS

The HIV protease inhibiting properties and the cell protective effect against HIV pathogenesis of the compounds of formula 1, or a therapeutically acceptable salt thereof, can be demonstrated by biochemical, microbiological and biological procedures.

A particular useful procedure for demonstrating the HIV protease inhibiting properties of the compounds of formula 1 or their therapeutically acceptable salts is the "Recombinant HIV Protease HLPC Assay". The procedure is based on the capacity of the test compound to inhibit enzymatic cleavage by HIV protease of a decapeptide (the substrate) having an amino acid sequence which includes a known HIV protease cleavage site of a HIV polyprotein; see H.G. Krausslich et al., Proc. Natl. Acad. Sci. USA, 86, 807 (1989). Details of this assay together with the results obtained for exemplified compounds of formula 1 are described in the examples hereinafter.

The capacity of the compounds of formula 1 or their therapeutically acceptable salts to protect cells against HIV infection can be demonstrated by microbiological procedures for evaluating the inhibitory effect of a test compound on the cytopathogenicity of HIV in human T4 cell lines. Typical of such procedures are those described by S. Harada and N. Yamamoto, Jpn. J. Cancer Res. (Gann), 76, 432 (1985), and S. Harada et al., Science, 229, 563 (1985). An assay based on the latter procedures is described in the examples hereinafter.

When a compound of this invention, or a therapeutically acceptable salt thereof, is used to combat HIV infections in a human, the peptide can be administered orally, topically or parenterally, in a vehicle comprising one or more pharmaceutically acceptable carriers, the proportion of which is determined by the solubility and chemical nature of the compound, the chosen route of administration and standard biological practice. For oral administration, the compound or a therapeutically acceptable salt thereof can be formulated in unit dosage forms such as capsules or tablets each containing a predetermined amount of the active ingredient, ranging from about 25 to 500 mg, in a pharmaceutically acceptable carrier. For topical administration, the compound can be formulated in a pharmaceutically acceptable vehicle containing 0.1 to 10 percent, preferably 0.5 to 5 percent, of the active agent. Such formulations can be in the form of a cream, lotion, sublingual tablet, or preferably a transdermal patch or buccal patch. For parenteral administration, the compound of formula 1 is administered by either intravenous, subcutaneous or intramuscular injection, in compositions with pharmaceutically acceptable vehicles or carriers. For administration by injection, it is preferred to use the compound in solution in a sterile aqueous vehicle which may also contain other solutes such as buffers or preservatives as well as sufficient quantities of pharmaceutically acceptable salts or of glucose to make the solution isotonic.

Suitable vehicles or carriers for the above noted formulations can be found in standard pharmaceutical texts, e.g. in "Remington's Pharmaceutical Sciences", 16th ed., Mack Publishing Company, Easton, Penn., 1980.

The dosage of the compound will vary with the form of administration and the particular active agent chosen. Furthermore, it will vary with the particular host under treatment. Generally, treatment is initiated with small dosages substantially less than the optimum dose of the peptide. Thereafter, the dosage is increased by small increments until the optimum effect under the circumstances is reached. In general, the compound is most desirably administered at a concentration level that will generally afford antivirally effective results without causing any harmful or deleterious side effects.

For oral administration, the compound or a therapeutically acceptable salt is administered in the range of 1.0 to 75 mg per kilogram of body weight per day, with a preferred range of 2.5 to 20 mg per kilogram. With reference to systemic administration, the compound of formula 1 is administered at a dosage of 10 mcg to 1000 mcg per kilogram of body weight per day, although the aforementioned variations will occur. However, a dosage level that is in the range of from about 50 mcg to 500 mcg per kilogram of body weight per day is most desirably employed in order to achieve effective results.

Although the formulations disclosed hereinabove are effective and relatively safe medications for treating HIV infections, the possible concurrent administration of these formulations with other antiviral medications or agents to obtain beneficial results is not excluded. Such other antiviral medications or agents include soluble CD4, zidovudine, dideoxycytidine, phosphonoformate, ribavarin or antiviral interferons (e.g. α-interferon or interleukin-2).

The following examples illustrate further this invention. Solution percentages or ratios express volume to volume relationship, unless stated otherwise. Temperatures are given in degrees Celsius. Proton nuclear magnetic resonance (NMR) spectra were obtained at 200 MHz. Abbreviations used in the examples include Boc: t-butyloxycarbonyl; BOP: (benzotriazol-1-yloxy)tris(dimethylamino)phosphonium hexafluorophosphate; Bzl: benzyl; DMF: dimethyl formamide; Et$_2$O: diethyl ether; EtOAc: ethyl acetate; EtOH: ethanol; HPLC: high performance liquid chromatography: MeOH: methanol; TFA: trifluoroacetic acid; THF: tetrahydrofuran; TLC: thin layer chromatography; Z: benzyloxycarbonyl.

Example 1

Boc-PheΨ[CH$_2$NH]Leu-Glu-NHCH$_2$CH$_2$CH$_2$Ph

A) Boc-Glu(OBzl)-NHCH$_2$CH$_2$CH$_2$Ph

N-methylmorpholine (3.3 mL, 5.5 mmol), the substituted amine 3-phenylpropylamine (0.78 mL, 5.5 mmol) and BOP (2.43 g, 5.5 mmol) were added to a solution of Boc-Glu(OBzl)-OH (1.69 g, 5.0 mmol) in acetonitrile (50 mL). The mixture was stirred at room temperature (20-22°) for 1 h, concentrated under reduced pressure to one half of its initial volume, and then diluted with EtOAc (50 mL). The resulting mixture was washed successively with cold 1 M aqueous HCl (3 x 15 mL), saturated aqueous NaHCO$_3$ (2 x 15 mL) and saturated brine (20 mL). The organic layer was dried (Na$_2$SO$_4$) and concentrated under reduced pressure. The residue was dissolved in warm EtOAc. The solution was diluted with 150 mL of Et$_2$O. The latter solution was washed several times with H$_2$O and then with a saturated aqueous solution of NaCl. The organic layer was dried (Na$_2$SO$_4$) and then concentrated under reduced pressure. The residue was crystallized from EtOAc-hexane to give the desired product as a white solid (1.43 g, 63%). $^1$H NMR (CDCl$_3$) δ 1.42 (9H, s), 1.81 (3H, complex m), 2.10 (1H, complex m), 2.42-2.55 (2H, complex m), 2.64 (2H, t, J = 7.1 Hz), 3.27 (2H, q, J = 6.8 Hz), 4.08 (1H, q, J = 5.6 Hz), 5.13 (2H, s), 5.17 (1H, broad m), 6.15 (1H, broad m), 7.15-7.35, (10H, complex m). $^1$H NMR also indicated that the product contained 5 mole percent of hexamethylphosphoric triamide.

B) Boc-PheΨ[CH$_2$NH]Leu-OH p-nitrobenzyl ester

Sodium cyanoborohydride (2.83 g. 45.0 mmol) was added portionwise over 15 min to a stirred solution of H-Leu-OH p-nitrobenzyl ester hydrochloride (10.36 g, 36.3 mmol) and Boc-phenylalaninal (15.06 g, 70.0 mmol), described by D.H. Rich and E.T.O. Sun, supra, in MeOH (250 ml). The resulting mixture was stirred for 18 h. The reaction mixture was added dropwise to a stirred, ice-cold saturated aqueous solution of NaHCO$_3$ (2.5 L). The aqueous solution was extracted twice with EtOAc. The combined organic extracts were washed three times with a saturated aqueous solution of NaCl, dried (MgSO$_4$) and concentrated. The residue was triturated with hexane to give a solid. The solid was purified by chromatography on silica gel (eluent: EtOAc-hexane, 1:4) to give the desired product as a white solid (12.33 g, 76%). $^1$H NMR (CDCl$_3$), 0.85-0.95 (6H, m), 1.3-1.85 (12H, m), 2.3-2.75 (12H, m), 2.75-2.9 (2H, m), 3.7-3.9 (1H, m), 4.5-4.7 (1H, m), 5.2 (2H, s), 7.1-7.4 (5H, m), 7.45-8.25 (4H, 2d).

C) Boc-PheΨ[CH$_2$NH]Leu-OH

The latter product (2.55 mg, 0.569 mmol) was dissolved in MeOH (5 ml). Oxygen was removed from the solution with a stream of dry nitrogen. Under an atmosphere of nitrogen, 10% palladium on charcoal (25 mg) was added to the solution. The mixture was shaken on a Parr hydrogenator under an atmosphere of hydrogen for 4h. The mixture was filtered through diatomaceous earth and the filtrate was concentrated to dryness. Trituration of the residue with Et$_2$O gave the first fragment Boc-PheΨ[CH$_2$NH]Leu-OH as a white solid (77.9 mg, 38%). $^1$H NMR (MeOH-d$_4$) δ 1.00 (6H, t, J = 6.0 Hz), 1.40 (9H, s), 1.56-1.84 (3H, complex m), 2.80-3.06 (3H, complex m), 3.20 (1H, dd, J$^1$ = 3.8 Hz, J$^2$ = 12.9 Hz), 3.54 (1H, t, J = 6.9 Hz), 4.05 (1H, m), 7.28 (5H, m).

D) Boc-PheΨ[CH$_2$NH]Leu-Glu(OBzl)-NHCH$_2$CH$_2$CH$_2$Ph

A solution of Boc-Glu(OBzl)-NHCH$_2$CH$_2$CH$_2$Ph (120 mg, 0.264 mmol) in 6 N HCl/dioxane (3 mL) was stirred at room temperature for 30 min. The solvent was removed under reduced pressure and the residue was dried in high vacuum (15 min). The residue was dissolved in acetonitrile (5 mL) and DMF (1 mL). N-methylmorpholine (174µL, 1.58 mmol), Boc-PheΨ[CH$_2$NH]Leu-OH (96.2 mg, 0.264 mmol) and BOP (128 mg, 2.90 mmol) were added to the solution. The mixture was stirred for 1 h at room temperature. Dimethylsulfoxide (0.5 mL) was added and the mixture was stirred for an additional 30 min. The reaction mixture was diluted with EtOAc (30 mL), washed with cold 1 M aqueous HCl (2 x 10 mL), saturated aqueous NaHCO$_3$ (2 x 10 mL) and saturated brine. The organic layer was separated, dried (Na$_2$SO$_4$) and

9

concentrated under reduced pressure. The residue was purified by chromatography on TLC grade silica gel (eluent: EtOAc-hexane, 3:2) to give the desired compound as a white solid (131 mg, 71%). $^1$H (CDCl$_3$) δ 0.90 (6H, t, J = 6.4 Hz), 1.37 (9H, s), 1.51-2.20 (7H, complex m), 2.35-2.64 (7H, complex m), 2.79 (2H, d, J = 6.3 Hz), 3.10-3.29 (3H, complex m), 3.87 (1H, broad q, J = 5.7 Hz), 4.37 (1H, q, J = 5.8 Hz), 4.83 (1H, d, J = 7.6 Hz), 5.12 (2H, s), 6.47 (1N, t, J = 5.4 Hz), 7.13-7.34 (15H, complex m), 7.82 (1N, d, J = 7.4 Hz).

E) Title compound of this example

The latter product (132 mg, 0.18 mmol) in a solution of MeOH (10 mL) was subjected to hydrogenolysis for 1h, using 20% Pd(OH)$_2$/C as catalyst. (Cf. section C of this example.) The reaction mixture was filtered through a 45µm membrane and the filtrate was concentrated. The residue was triturated with Et$_2$O to give Boc-PheΨ[CH$_2$NH]-Leu-Glu-NHCH$_2$CH$_2$CH$_2$Ph as a white solid (108 mg, 94%). $^1$H NMR (CDCl$_3$) δ 0.92 and 0.97 (6H, d, J = 6.0 Hz), 1.40 (9H, s), 1.60-2.11 (9H, complex m), 2.38 (2N, t, J = 7.1 Hz), 2.65 (2H, t, J = 8.0 Hz), 2.84 (4N, t, J = 6.3 Hz), 3.31 (2H, m), 3.94 (1H, m), 4.40 (1H, dd, J = 5.7 Hz), 7.16-7.74 (10H, complex m), 8.06 (1H, t, J = 5.8 Hz).

Example 2

Boc-PheΨ[CH$_2$NH]Cpa-Glu-NHCH$_2$CH$_2$CH$_2$Ph

By following the procedure of example 1 but substituting H-Cpa-OH p-nitrobenzyl ester hydrochloride for H-Leu-OH p-nitrobenzyl ester hydrochloride, the title compound was obtained. Mass spectrum: 609 (M + H)$^+$, 631 (M + Na)$^+$. Amino acid analysis: Glu, 1.0; phenylpropylamine, present (PheΨ[CH$_2$NH]Cpa does not hydrolyze).

Example 3

(C$_2$H$_5$)$_2$CHC(O)-PheΨ[CH$_2$NH]Cpa-Glu-NHCH$_2$CH$_2$CH$_2$Ph

By following the procedure of example 1 but substituting (C$_2$H$_5$)$_2$CHC(O)-phenylalaninal for Boc-phenylalaninal, the title compound was obtained. Mass spectrum: 607 (M + H)$^+$, 629 (M + Na)$^+$. Amino acid analysis: Glu, 1.0; phenylpropylamine, present (PheΨ[CH$_2$NH]Cpa does not hydrolyze).

By following the procedure of example 1 and by substituting the appropriate first fragment (e.g. Boc-A-OH wherein A is as defined herein), and/or by substituting the appropriate amine or alcohol for the substituted amine in section A of example 1, the following compounds of formula 1 were obtained:

PhCH$_2$C(O)-TyrΨ[CH$_2$NH]Leu-Glu-NHCH$_2$CH$_2$CH$_2$-(cyclohexyl)

Boc-PheΨ[CH$_2$NH]Leu-NHCH(CH$_2$CH$_2$CH$_2$COOH)C(O)-NHCH$_2$CH$_2$CH$_2$Ph

Boc-PheΨ[CH$_2$NH]Leu-Glu-OCH$_2$CH$_2$CH$_2$Ph

Boc-PheΨ[CH$_2$NH]Cpa-Glu-OCH$_2$CH$_2$CH$_2$Ph

(C$_2$H$_5$)$_2$CHC(O)-CpaΨ[CH$_2$NH]Phe-Glu-OCH$_2$CH$_2$CH$_2$CH$_3$

Example 4

Boc-ACHPA-Glu-NHCH$_2$CH$_2$CH$_2$Ph

A solution of the protected amide Boc-Glu(OBzl)-NHCH$_2$CH$_2$CH$_2$Ph (618.5 mg, 1.36 mmol), described in example 1, in 6N HCl/dioxane (10 mL) was stirred at room temperature under a nitrogen atmosphere for 15 min. The solvent was evaporated and the residue was suspended in dry acetonitrile (30 ml). Under a nitrogen atmosphere, the suspension was adjusted to pH 8 (wet pH paper) by the addition of N-methylmorpholine (excess), then the first fragment Boc-ACHPA-OH (429 mg, 1.36 mmol) and BOP (661 mg, 1.50 mmol) were added. The mixture was stirred at room temperature for 2h. The mixture was poured into a saturated aqueous solution of NaCl. The aqueous solution was extracted twice with EtOAc. The combined organic extracts were washed successively with 5% aqueous HCl (two times), saturated aqueous NaHCO$_3$ - (two times) and saturated aqueous NaCl (two times). The organic extract was dried (Na$_2$SO$_4$) and evaporated to dryness. Chromatography of the residue on silica gel (30g), using a gradient of 1 to 5% MeOH in CHCl$_3$, afforded a crude product (620 mg) which comprised the desired intermediate, Boc-ACHPA-Glu(OBzl)-NHCH$_2$CH$_2$CH$_2$Ph, contaminated with some hexamethylphosphoric triamide (as indicated by NMR). By subjecting the crude product to chromatography on silica gel (25 g) using EtOAc as the eluent, the pure intermediate was obtained as an oil (580 mg, 53%). $^1$H NMR (CDCl$_3$) $\delta$ 1.1-1.6 (11H, m), 1.4 (2H, s), 1.8 (2H, m), 2.3-2.6 (4H, m), 2.6 (2H, t), 3.5-3.6 (2H, m), 4.0 (1H, m), 4.4 (1H, m), 4.55 (1H, d), 5.1 (2H, s), 6.7 (2H, m), 7.1-7.4 (10H, m).

The intermediate (80 mg, 0.10 mmol) was dissolved in MeOH (20 mL) and subjected to hydrogenolysis using 20% Pd(OH)$_2$ on carbon as the catalyst. The reaction mixture was processed in the usual manner (see section C of example 1) to give the title compound as an oil (36 mg, 53%). Mass spectrum: 562 (M + H)$^+$. Amino acid analysis: Glu, 1.00; ACHPA, 1.05. $^1$H NMR (CDCl$_3$) $\delta$ 1.42 (9H, s), 0.5-2.75 (23H, complex broad m), 3.20 (2H, broad m), 3.59 (2H, broad m), 3.94 (1H, broad m), 4.46 (1H, broad m), 5.11 (1H, broad m), 3.5-5.2 (1H, broad m), 6.95-7.32 (5H, m), 7.32-7.80 (2H, broad m).

Example 5

Boc-ACHPA-Glu(OMe)-NHCH$_2$CH$_2$CH$_2$Ph

The title compound of example 4 (18 mg) was dissolved in dry Et$_2$O (10 mL). A slight excess of diazomethane in dry Et$_2$O was added to the solution (as indicated by the mixture maintaining a yellow color). The mixture was evaporated to dryness and the residual oil was dried under high vacuum to afford a quantitative yield of the title compound. Mass spectrum: 576 (M + H)$^+$. Amino acid analysis: Glu, 1.00; ACHPA, 1.00.

Example 6

(C$_2$H$_5$)$_2$CHC(O)-ACHPA-Glu-NHCH$_2$CH$_2$Ph

For the following preparation, Boc-Glu(OBzl)-NHCH$_2$CH$_2$Ph was prepared in the same manner as described for Boc-Glu(OBzl)-NHCH$_2$CH$_2$CH$_2$Ph in example 1 except that 2-phenylethylamine was substituted for 3-phenylpropylamine.

A solution of Boc-Glu(OBzl)-NHCH$_2$CH$_2$Ph (1.32 g, 3.0 mmol) in 6 N HCl/dioxane (10 ml) was stirred under a nitrogen atmosphere at room temperature for 15 min. The solvent was evaporated and the residue was dried under high vacuum. The solid residue was suspended in dry acetonitrile (30 mL). Under a nitrogen atmosphere, the stirred suspension was ajusted to pH 8 (wet pH paper) by the addition of N-methylmorpholine, then Boc-ACHPA-OH (1.32 g, 3.0 mmol) and BOP (1.46 g, 3.3 mmol) were added. The mixture was stirred at room temperature for 2h.

The mixture was poured into a saturated aqueous solution of NaCl. The aqueous solution was extracted twice with EtOAc. The combined organic extracts were washed successively with 5% aqueous HCl (two times), saturated aqueous NaHCO$_3$ (two times) and saturated aqueous NaCl (two times). Thereafter, the extract was dried (Na$_2$SO$_4$) and concentrated to dryness. Chromatography of the residual oil on silica gel (30 g), using a gradient of 1 to 5% MeOH in CHCl$_3$, afforded Boc-ACHPA-Glu(OBzl)- NHCH$_2$CH$_2$Ph (1.1 g, 57%). A solution of the latter compound (159 mg, 0.25 mmol) in 6 N HCl/dioxane (3 mL) was stirred at room temperature under a nitrogen atmosphere for 15 min. The solvent was evaporated and the residue was suspended in dry acetonitrile (10 mL). Under a nitrogen atmosphere, the stirred suspension was ajusted to pH 8 (wet pH paper) by the addition of N-methylmorpholine, then 2-ethylbutyric acid (31 L, 0.25 mmol) and BOP (121.6 mg, 0.275 mmol) were added. The solution was stirred at room temperature for 2h. The mixture was poured into a saturated aqueous solution of NaCl. The aqueous solution was extracted twice with EtOAc. The combined organic extracts were washed successively with 5% aqueous HCl (two times), saturated aqueous NaHCO$_3$ (two times) and saturated aqueous NaCl (two times). Thereafter, the organic extract was dried (Na$_2$SO$_4$) and concentrated to dryness. Chromatography of the residue on silica gel (20 g), eluting with a 1 to 5% gradient of MeOH in CHCl$_3$ afforded a crude product. The crude product was subjected to chromatography on TLC grade silica gel (10 g), using EtOAc as the eluent, to afford pure (C$_2$H$_5$)$_2$CHC(O)-ACHPA-Glu(OBzl)NHCH$_2$CH$_2$Ph.

The latter compound was subjected hydrogenolysis in the same manner as described for the hydrogenolysis in example 1. The usual processing of the reaction mixture afforded an oil which on trituration with hexane afforded the title compound as a white solid (27.1 mg, 20%). Mass spectrum: 546 (M + H)$^+$. Amino acid analysis: Glu, 1.00; ACHPA, 1.04.

The following compounds of formula 1 were prepared by following the procedure of example 4, 5 or 6 and by using the appropriate first fragment (e.g. Boc-A-OH wherein A is as defined herein), and/or by substituting the appropriate protected ester [e.g. Boc-NHCH(R$^4$)C(O)-Y wherein R$^4$ and Y are as defined herein] for the corresponding protected amide, see example 4.

11

Example 7:
Boc-ACHPA-Glu-NHCH$_2$CH$_2$Ph, mass spectrum: 548 (M + H)$^+$, amino acid analysis: Glu, 1.03; ACHPA, 0.98; phenethylamine, 1.01.

Example 8:
C$_2$H$_5$C(CH$_3$)$_2$C(O)-ACHPA-Glu-NHCH$_2$CH$_2$Ph, mass spectrum: 546 (M + H)$^+$, amino acid analysis: Glu, 1.00; ACHPA, 1.03.

Example 9:
CH$_3$C(CH$_3$)$_2$CH$_2$C(O)-ACHPA-Glu-NHCH$_2$CH$_2$Ph, mass spectrum: 546 (M + H)$^+$, amino acid analysis: Glu, 1.00; ACHPA, 1.08.

Example 10:
CH$_3$CH$_2$CH$_2$C(O)-ACHPA-Glu-NHCH$_2$CH$_2$Ph, mass spectrum: 518 (M + H)$^+$, amino acid analysis: Glu, 1.04: ACHPA, 0.99: phenethylamine, 0.98.

Example 11:
CH$_3$CH(CH$_3$)CH$_2$C(O)-ACHPA-Glu-NHCH$_2$CH$_2$Ph, mass spectrum: 532 (M + H)$^+$, amino acid analysis: Glu, 1.04; ACHPA, 0.97; phenethylamine, 0.99.

Example 12:
(Cyclohexylcarbonyl)-ACHPA-Glu-NHCH$_2$CH$_2$Ph, mass spectrum: 558 (M + H)$^+$, amino acid analysis: Glu, 1.04: ACHPA, 0.98; phenethylamine, 0.98.

Example 13:
Boc-ACHPA-Glu-NH(CH$_2$)$_7$CH$_3$, mass spectrum: 556 (M + H)$^+$ and 578 (M + Na)$^+$, amino acid analysis: Glu, 1.08; ACHPA, 0.92.

Example 14:
(C$_2$H$_5$)$_2$CHC(O)-ACHPA-Glu-NHCH$_2$CH$_2$CH$_2$Ph, mass spectrum: 560.5 (M + H)$^+$, amino acid analysis: Glu, 1.00; ACHPA, 1.08.

Example 15:
Boc-ACHPA-Glu-NHCH$_2$CH$_2$CH$_2$-(cyclohexyl), mass spectrum: 568 (M + H)$^+$ and 590 (M + Na)$^+$, amino acid analysis: Glu, 1.09, ACHPA, 0.91.

Example 16:
Boc-ACHPA-Glu(OMe)-NHCH$_2$CH$_2$CH$_2$-(cyclohexyl), mass spectrum: 582 (M + H)$^+$ and 604 (M + Na)$^+$, amino acid analysis: Glu, 1.00, ACHPA, 0.70.

Example 17:
Boc-NorSta-Glu-NHCH$_2$CH$_2$CH$_2$Ph, amino acid analysis: Glu, 1.00; NorSta, 1.00; mass spectrum: 522 (M + H)$^+$ and 544 (M + Na)$^+$.

Example 18:
Boc-ACPPA-Glu-NHCH$_2$CH$_2$CH$_2$Ph, mass spectrum: 520 (M + H)$^+$, amino acid analysis: Glu, 1.00; ACPPA, 1.00.

Example 19:
Boc-ACHPA-Glu-NHCH$_2$CH$_2$-[4NH$_2$SO$_2$-Ph], mass spectrum: 627 (M + H)$^+$, 649 (M + Na)$^+$, amino acid analysis: Glu, 1.06; ACHPA, 0.96; 4-(aminosulfonyl)phenylethylamine, 0.98.

Example 20:
Boc-ACHPA-Glu-NHCH$_2$CH$_2$CH$_2$-(4HO-Ph), mass spectrum: 600 (M + Na)$^+$, amino acid analysis: Glu, 1.05; ACHPA, 0.95.

Example 21:
Boc-ACHPA-Glu-NHCH$_2$CH$_2$-(4F-Ph), mass spectrum: 566 (M + H)$^+$ and 588 (M + Na)$^+$, amino acid analysis:

Glu, 1.0; ACHPA, 1.06; 4-fluorophenylethylamine, 1.36.

Example 22:
Boc-ACHPA-Glu-NHCH$_2$CH$_2$-(4MeS-Ph), mass spectrum: 594 (M+H)$^+$ and 616 (M+H)$^+$, amino acid analysis: Glu, 1.06; ACHPA, 0.94; 4-(methylthio)phenylethylamine, present.

Example 23:
Boc-ACHPA-Glu-NHCH$_2$CH$_2$CH$_2$-(4PrO-Ph), mass spectrum: 620 (M+H)$^+$ and 642 (M+Na)$^+$, amino acid analysis: Glu, 1.08; ACHPA, 0.92.

Example 24:
Moc-ACHPA-Glu(OMe)-NHCH$_2$CH$_2$CH$_2$Ph, mass spectrum: 534 (M+H)$^+$ and 556 (M+Na)$^+$.

Example 25:
Boc-ACHPA-Glu-OCH$_2$CH$_2$CH$_2$Ph, mass spectrum: 563 (M+H)$^+$ and 585 (M+Na)$^+$ amino acid analysis: Glu, 1.00; ACHPA, present: phenylpropylamine, 1.04.

Example 26:
(CH$_3$)$_3$CNHCO-ACHPA-Glu-NHCH$_2$CH$_2$CH$_2$Ph, mass spectrum: 561 (M+X)$^+$ and 583 (M+Na)$^+$, amino acid analysis: Glu, 1.0; ACHPA, present: propylamine, 1.04

Still other examples of compounds of formula 1 which can be prepared by the aforementioned procedures are:

Example 27:
Boc-ACHPA-Glu-OCH$_2$CH$_2$-(4F-Ph)

Example 28:
CH$_3$CH$_2$C(CH$_3$)$_2$C(O)-ACHPA-Glu(OMe)-OCH$_2$CH$_2$CH$_2$Ph

Example 29:
Boc-PheΨ[CH$_2$NH]Cpa-Glu(OMe)-NHCH$_2$CH$_2$CH$_2$Ph

Example 30:
(C$_2$H$_5$)$_2$CHC(O)-PheΨ[CH$_2$NH]Cpa-Glu(OMe)-NHCH$_2$CH$_2$Ph

Example 31

Boc-ACHPA-GluΨ[CSNH]-NHCH$_2$CH$_2$CH$_2$Ph

A) Boc-Glu(OBzl)Ψ[CSNH]-NHCH$_2$CH$_2$CH$_2$Ph

A stirred mixture of Boc-Glu(OBzl)-NHCH$_2$CH$_2$CH$_2$Ph (227 mg, 0.5 mmol), described in Example 1, and Lawesson's reagent, [2,4 bis(4-methoxyphenyl)-1,3,2,4-dithiaphosphetane 2,4-disulfide, 120 mg, 0.3 mmol], see K. Clausen et al., Chemica Scripta, 20, 14 (1982), in dry THF (5 ml) was heated at 60° for 15 min. The reaction solvent was evaporated under reduced pressure. The resulting yellow oil was subjected to chromatography on TLC grade silica gel (30 g), using EtOAc-hexane (3:7) as the eluent. The desired thioamide, i.e. the title compound of this section, was thus obtained as a colorless oil (202 mg, 86%). $^1$H NMR (CDCl$_3$) δ 1.42 (9H, s), 1.88-2.16 (4H, complex m), 2.31-2.59 (2H, m), 2.67 (2H, t, J = 7.3 Hz), 3.65 (2H, q, J = 6.7 Hz), 4.38 (1H, q, J = 6.1 Hz), 5.12 (2H, s), 5.53 (1H, d, J = 8.3 Hz), 7.15-7.34 (10H, m), 8.07 (1H, broad, m).

B) Boc-ACHPA-Glu(OBzl)Ψ[CSNH]-NHCH$_2$CH$_2$CH$_2$Ph

The title compound of section A) of this example (200 mg, 0.43 mmol) was coupled with Boc-ACHPA-OH (135 mg, 0.43 mmol), using BOP (228 mg 0.51 mmol), according to the procedure described in Example 4, to give the title compound of this section (182 mg, 63%) after purification by chromatography on silica gel (eluent = EtOAc-hexane, 1:1).

C) The title compound of this example

A 1M aqueous solution of NaOH (354$\mu$L, 0.35 mmol) was added to a solution of the title compound of section B) of this example (182 mg, 0.27 mmol) in MeOH (4.5 mL) and $H_2O$ (2.5 mL). After standing at room temperature for 1 h, additional MeOH (10 mL) was added to the reaction mixture. The mixture was heated at 60° for 1 h. The mixture was concentrated to one half of its original volume, diluted with $H_2O$ (10 mL) and extracted with $CH_2Cl_2$ (3 x 5 mL). The extract was dried ($Na_2SO_4$) and concentrated to dryness. Chromatrography of the residual oil on silica gel (20 g), using a gradient of 1 to 20% MeOH in $CHCl_3$, afforded a white solid. The solid was triturated with pentane to afford the title compound of this example as a white solid (74.3 mg, 47%). $^1H$ NMR ($CDCl_3$, Me ester, $CH_2N_2$-$Et_2O$) $\delta$ 0.8-0.84 (2H, m), 1.14-1.34 (5H, complex m), 1.44 (9H, s), 1.54-1.80 (6H, broad m), 1.91-2.19 (4H, complex m), 2.28-2.55 (4H, complex m), 2.67 (2H, t, J = 8.1 Hz), 3.67 (3H, s), 3.58-3.72 (3H, m), 3.98 (1H, m), 4.70-4.77 (2H, m), 7.16-7.33 (6H, m), 8.80 (1H, broad m).

Analogous thioamides of formula 1 can be prepared by selecting the appropriate reactants and following the procedure of this example. Examples of such thioamides are:

Boc-Phe$\Psi$[$CH_2$NH]Leu-Glu$\Psi$[CSNH]-NHCH$_2$CH$_2$CH$_2$Ph

Boc-ACHPA-Glu(OMe)$\Psi$[CSNH]–NHCH$_2$CH$_2$Ph

Boc-ACHPA-Glu$\Psi$[CSNH]-NH(CH$_2$)$_5$CH$_3$

Bzl-C(O)-Phe$\Psi$[$CH_2$NH]Cpa-Glu$\Psi$[CSNH]-NHCH$_2$CH$_2$CH$_2$Ph

Example 32

4-{     [4$\beta$(R),5$\beta$(S)]-5-cyclohexyl-2,2-dimethyl-3-(2,2-dimethylethoxycarbonyl)oxazolidine}-(R,S)-$\beta$-hydroxypropanoic Acid [Intermediate for the compound of formula 1 in which A is (S,R,S)-NHCH[CH(OH)-cyclohexyl]CH(OH)CH$_2$C(O), i.e. A is HACHPA.

A mixture of (2RS,3S)-PhCH(OH)CH(NHZ)COOEt (30g), see H. Umezawa et al., Ger. Offen. 2,632,396, February 3, 1977, also Chem. Abst., 87, 23736e (1977), crude freshly ground papain (90g, 1 IU/mg, Sephadex ® G-50 (90 g) and 0.3 M potassium citrate buffer (150 mL, pH 7) was incubated for 45 min at 37°. A solution of 18 crown-6 ether (11.88 g) in EtOH (750 mL) was added and the resultant mixture was stirred at 37° for 4 days. The reaction was followed by TLC (MeOH-AcOH-CHCl$_3$, 10:1:89). The mixture was filtered and the filtrate concentrated to dryness. The residue was purified by chromatography (SiO$_2$: MeOH-AcOH-CH$_2$Cl$_2$, 10:1:189) to give (2S,3S)-PhCH(OH)CH(NHZ)COOH (6.3 g).

The latter product (6.3 g) was reacted with excess diazomethane in Et$_2$O solution. At the end of the reaction, excess diazomethane was consumed with AcOH and the resulting mixture was evaporated to dryness. The residue was purified by chromatography (SiO$_2$, eluent: EtOAc-hexane, 1:4: followed by EtOAc) to give (2S,3S)-PhCH(OH)CH(NHZ)COOCH$_3$ (4.7 g).

The latter product (4.2 g) was dissolved in THF (150 mL). (Boc)$_2$O (3.0 g) and 10% palladium on charcoal were added the solution. The mixture was shaken on a Parr hydrogenator under one atmosphere of hydrogen for 18 h, an additional amount (100 mg) of 10% palladium on charcoal being added to the mixture after the first hour. The mixture was filtered through diatomaceous earth and the filtrate concentrated to dryness. The residue was purified by chromatography (SiO$_2$, eluent: EtOAc-CH$_2$Cl$_2$, 1:9) to give (2S,3S)-PhCH(OH)CH(NHBoc)COOCH$_3$ (3.5 g).

The latter product (3.5 g) was dissolved in MeOH (70 mL). Rh/Al$_2$O$_3$ (700 mg) was added to the solution. The mixture was shaken on a Parr hydrogenator under hydrogen (45 psi) for 2 1/2 days. Filtration of the mixture and evaporation of the filtrate gave (2S,3S)-cyclohexyl-CH(OH)CH(NHBoc)COOCH$_3$ (3.7 g).

A solution of the latter product (3.7 g), 2,2-dimethoxypropane (3 mL), p-toluenesulfonic acid (30 mg) in benzene (75 mL) was heated at reflux in a Dean-Stark reflux apparatus for 6h, an additional amount (1 mL) of 2,2-dimethoxypropane being added to the mixture after the first 2h. The mixture was concentrated, diluted with Et$_2$O, washed with 10% aqueous NaOAc and brine, dried (MgSO$_4$) and concentrated to dryness. The residue was purified by chromatography (SiO$_2$, eluent: EtOAc-hexane, 4:1) to give the corresponding O,N-acetonide (3.5 g), i.e. 4-{[4$\beta$(S),5$\beta$(S)]-5-cyclohexyl-2,2-dimethyl-3-(2,2-dimethylethoxycarbonyl)-oxazolidine]carboxylic acid methyl ester.

A solution of the latter O,N-acetonide (2.0 g) in THF (20 mL) was cooled to 0°. Lithium aluminum hydride (0.50 g) was added in small portions to the cooled solution. After 1h at 0°, diatomaceous earth was added to the reaction mixture to render it neutral and excess lithium aluminum hydride was decomposed by the dropwise addition of H$_2$O at 0°. The resultant mixture was filtered . The collected matter on the filter was washed with EtOAc and H$_2$O. The combined filtrate and washing were poured into a separatory funnel. The organic phase was separated, dried (MgSO$_4$), filtered and concentrated to afford the corresponding alcohol, i.e. 4-{ [4$\beta$(R),5$\beta$(S)]-5-cyclohexyl-2,2-dimethyl-3-(2,2-dimethylethoxycarbonyl)-oxazolidine} methanol (1.77 g), $^1$H NMR(CDCl$_3$) $\delta$ 3.7 (2H, m), 3.85 (1H, m), 4.1 (1H, m).

The latter alcohol was oxidized to the corresponding aldehyde and then alkylated as follows. Under an anhydrous N$_2$ atmosphere, a solution of oxalyl chloride (500 $\mu$L, 1.1 equiv.) in CH$_2$Cl$_2$ (20 mL) was cooled to -70$^\circ$C. Dimethylsulfoxide (475 $\mu$L, 1.3 equiv.) was added to the cooled solution. After 15 min., a solution of the above noted alcohol (1.77 g) in CH$_2$Cl$_2$ (8 mL) was added dropwise. After a further 90 min. at -70$^\circ$, diisopropylethylamine (4.5 ml, 5 equiv.) was added to the mixture and the resultant solution was allowed to stand at -70$^\circ$C for a further 90 min. To this latter solution (i.e. the first solution), a second solution was added via a cannula while maintaining a reaction temperature of < -55$^\circ$, the second solution having been prepared as follows: Under an anhydrous N$_2$ atmosphere, a solution of diisopropylamine (2.5 mL, 3.3 equiv.) in THF (15 mL) containing a trace of 2,2'-dipiridyl was cooled to -5$^\circ$. Butyllithium (10.5 mL of a 1.6 M solution in hexane, 3.3 equiv.) was added to the latter cooled solution. After 20 min., the mixture was cooled to -70$^\circ$ and EtOAc (1.5 mL, 3.0 equiv.) was added dropwise. After 1h, the resultant second solution was added by cannula to the first solution under anhydrous conditions and at low temperature. The mixture of the two solutions was kept at -60$^\circ$ for 30 min. Thereafter, a 10% aqueous solution of NH$_4$Cl was added. The mixture was extracted with EtOAc (2X). The extract was washed with brine, dried (MgSO$_4$), and evaporated to dryness. The residue was purified by chromatography (SiO$_2$, eluent: EtOAc-hexane, 3:17) to give the ester 4-{[4$\beta$(R),5$\beta$(S)]-5-cyclohexyl-2,2-dimethyl-3-(2,2-dimethylethoxycarbonyl)oxazolidine}-(R,S)-$\beta$-hydroxypropanoic acid ethyl ester (1.1 g).

A solution of the latter ester (1.2 g) in EtOH (100 mL) was cooled to 0$^\circ$. 1N aqueous NaOH (3.5 mL) was added dropwise to the cooled solution. The hydrolysis of the ester was followed by TLC (EtOAc-hexane, 1:4). After 3h, additional 1N aqueous NaOH (0.5 mL) was added. After another 3h, the reaction mixture was concentrated to a small volume, diluted with H$_2$O, and washed with Et$_2$O. The aqueous phase is acidified with 5% aqueous citric acid, extracted with EtOAc (3X), dried (MgSO$_4$) and concentrated to give the corresponding free acid (1.0 g), i.e. the title compound.

In the same manner, other intermediates for the compounds of formula 1 in which A is NHCH[CH(OH)-R$^{3A}$]CH(OH)CH$_2$C(O) wherein R$^{3A}$ is as defined herein by replacing (2S,3S)-cyclohexyl-CH(OH)CH(NHBoc)-COOCH$_3$ with the appropriately terminally substituted ester. The intermediate can be resolved at this stage or alternative the (S,R,RS) diastereoisomeric mixture can be transformed to the desired compound of formula 1 which in turn can be resolved. For instance, see the following example.

Example 33

Boc-HACHPA-Glu-NHCH$_2$CH$_2$CH$_2$Ph

A solution of Boc-Glu(OBzl)-NHCH$_2$CH$_2$CH$_2$Ph (200 mg), described in example 1, in CH$_2$Cl$_2$-TFA (1:1, 10 mL) was allowed to stand for 30 min at room temperature. Thereafter, the solution was concentrated under reduced pressure. Hexane was added to the residue and the mixture was concentrated to dryness under reduced pressure (2X) to give H-Glu(OBzl)-NHCH$_2$CH$_2$CH$_2$Ph. The latter product (1.0 equiv.) was dissolved in CH$_2$Cl$_2$ (15 mL). The title compound of example 32 (178 mg, 1.05 equiv.) and BOP (211 mg, 1.05 equiv.) were added to the solution. The solution was cooled to 0$^\circ$ and the pH of the solution was adjusted to 8 by the addition of diisopropylethylamine. After 90 min., 5% aqueous Na$_2$CO$_3$ wasadded and the resulting mixture was extracted with EtOAc. The extract was washed with 5% aqueous citric acid and brine, dried (MgSO$_4$) and concentrated to dryness to give the corresponding O,N-acetonide of [(S,R,RS)-cyclohexyl-CH(OH)CH(NHBoc)-CH(OH)CH$_2$C(O)]-Glu(OBzl)-NHCH$_2$CH$_2$CH$_2$Ph (323 mg).

A mixture of the latter product (322 mg), ($\pm$)-10-camphor-SO$_3$H (trace amount), H$_2$O (35 $\mu$L) and acetonitrile (3 mL) was heated at reflux for 5 1/2 h, additional amounts of ($\pm$)-10-camphor-SO$_3$H (trace) and H$_2$O (50 $\mu$L) being added after the first 2h. The mixture was cooled and extracted with EtOAc. The extract was washed with 10% aqueous NH$_4$OAc and brine, dried (MgSO$_4$) and evaporated to dryness. NMR examination of the crude product indicated that the reaction had cleaved the acetonide and partially removed the Boc protecting group. The crude product was subjected to hydrogenolysis for 2h, using 10% palladium on carbon as catalyst. The reaction mixture was filtered through diatomaceous earth and the filtrate evaporated to give a (S,R,RS)-isomeric mixture (228 mg) of the title compound and H-HACHPA-Glu-NHCH$_2$CH$_2$CH$_2$Ph. The latter crude product (228 mg) was dissolved in tert-butanol (5 mL). (Boc)$_2$O (76.9 mg) and 1N aqueous NaOH (0.355 ML) were added to the solution. The resulting mixture was allowed to stand at room temperature for 4h. Aqueous NaOH (3 drops) were added. The mixture was diluted with H$_2$O and washed with ET$_2$O. The aqueous phase was acidified with 5% aqueous citric acid and extracted with EtOAc. The latter extract was dried (MgSO$_4$) and evaporated to give a crude product (220 mg), a (S,R,RS)-isomeric mixture. The diasteroisomers were separated by HPLC using a Whatman Partisil®10 ODS-3 column (2.2 x 50 cm), 10 micron particle size. Two solutions were used for elution: solution A, 0.06% TFA in $H_2O$; and solution B, 0.06% TFA in acetonitrile. The elution gradient was 20% to 70% of solution B in 80 min.; flow rate = 20 mL/min. Two pure fractions, as determined by analytical HPLC, were obtained. The more polar isomer (25 mg) was the diastereoisomer in which a 3(R)-HACHPA was incorporated therein and the less polar isomer (70 mg) was the title compound of this example, mass spectrum: 578 $(M+H)^+$.

Example 34

Recombinant HIV Protease HPLC Assay:

Enzyme:

HIV protease was expressed in E. coli [construct pBRT1 prt[+], see W.G. Farmerie et al., Science, 236, 305 (1987)], according to the following protocol:
Unless stated otherwise, all solutions are aqueous solutions.

(i) Fermentation

E. coli cells containing the pBRT1 prt[+] plasmid were used to inoculate a seed culture media composed of Luria-Bertani Broth containing 100μg/mL of ampicillin. Flasks were incubated at 37° under agitation for 17 h. Production flasks containing sterile M9 broth, supplemented with 100μg/ml of ampicillin, were inoculated at a level of 1% (v/v) using the seed culture described above. Total volume in each production flask was 500 mL in a 2 L Erlenmeyer flask. Flasks were incubated at 37° under agitation until a cell concentration corresponding to an optical density ($\lambda$ = 540 m) of 0.6 was reached (no dilution). This time span was routinely 3-4 h. Flasks were then supplemented with 5mM isopropylthiogalactoside (IPTG, Research Organics, Cleveland, Ohio, USA) and incubation was continued until the cell concentration reached an optical density of 0.2 at 16-fold dilution. Flasks were then supplemented with 1mM phenylmethylsulfonyl fluoride (PMSF) and rapidly chilled to 4°. The bacterial cells were recovered by centrifugation at 4°. The wet pellet was stored at -70°.

(ii) Extraction and Preparation of Assay Grade Enzyme:

All steps below were performed at 4° unless otherwise indicated. Thawed cells were mixed with buffer A [50 mM tris(hydroxymethyl)aminoethane HCl(Tris-HCl, pH 7.4); 0.6 mM ethylenediaminetetraacetic acid (EDTA); 0.375 M NaCl, 0.2% Nonidet P-40 ® (BDH Chemicals Ltd., Poole, UK); 1mM PMSF], at a ratio of one part cells to nine parts buffer A. Diatomaceous earth (Celite 545 ® , John Manville, Lompoc, CA, USA) was added at a ratio of two parts to one part of wet cell weight. The resulting slurry was homogenized at high speed (ca. 20,000 rpm) on a Waring ® industrial blender for 8 x 15 sec. pulses. Cell debris/Celite ® was collected by centrifugation and the resulting pellet was extracted with 4.5 parts of buffer A to one part wet solids using the homogenization procedure described above. Supernatants from both homogenization steps were combined and soluble protein was precipitated by the addition of solid $(NH_4)_2SO_4$ to yield a final concentration of 75% saturation. This mixture was agitated for 60 min and the precipitate was recovered by centrifugation. The resulting pellet was suspended in buffer B [50mM Tris-HCl, pH 8; 30 mM NaCl; 1 mM DL-dithiothreitol(DTT); 1 mM EDTA; 1 mM PMSF; 10% glycerol], and dialyzed for 18h against the same buffer.

An aliquot of the dialyzed extract containing 150 mg of the protein was loaded onto a Sephadex A25 ® anion exchange column. (Pharmacia, Uppsala, Sweden) with bed dimensions of 70 cm length and 2.5 cm diameter. The sample was eluted isocratically with buffer B at a linear flow rate of 10 cm/h. Fractions containing HIV protease activity (see below for assay description) were combined, and soluble protein was precipitated by the addition of saturated aqueous $(NH_4)_2SO_4$ to yield a total $(NH_4)_2SO_4$ concentration of 85% saturation. Precipitated protein was removed by centrifugation and the resulting pellet was dissolved in buffer C [50 mM 2-(4-morpholino)ethanesulfonic acid (MES), pH 5.5; 150 mM NaCl; 1 mM DTT; 1 mM EDTA; 10% glycerol). This preparation was dialyzed for 18h against buffer C, and then frozen at -70°. All crude extracts were purified by chromatography in aliquots containing 150 mg of protein in the same manner as described above. The final preparations from each batch were pooled, divided into 34μL aliquots and stored at -70°. The final protein recovered from a 20 L fermentation was typically 300 mg with a specific activity for HIV protease of 18.2 mmoles of substrate cleaved/min/mg.

16

The aliquots were diluted to 1/38 of the original concentration with buffer, see below, prior to use (i.e. the enzyme working solution).

Substrate:

VSFNFPQITL-NH$_2$, MW 1164, see Krausslich et al., Proc. Natl. Acad. Sci. USA, 86, 807 (1989), was used as substrate. The substrate was made into 10 mM stock in DMSO and stored at $\overrightarrow{4}$. Prior to use, the stock was diluted with buffer to give a 400$\mu$M solution (i.e. the substrate working solution).

Buffer:

MES (100 mM), KCl (300 mM) and EDTA (5 mM) were dissolved in distilled H$_2$O (90 mL) and the solution was adjusted to pH 5.5 with concentrated aqueous NaOH. The latter solution was diluted to 100 mL with H$_2$O to give the buffer.

Procedure:

(1 ) The assay mixture was prepared by mixing 20$\mu$L of the substrate working solution, 10$\mu$L of the solution of the test compound in 10% DMSO and 10$\mu$L of the enzyme working solution. (2) The assay mixture was incubated at 37° for 30 min. (3) The reaction was quenched by adding 200$\mu$L of 2% aqueous TFA. (4) The substrate and products (i.e. VSFNF and PQITL-NH$_2$) were separated by subjecting 100$\mu$L of the quenched assay mixture to HPLC using a Perkin-Elmer 3 x 3 CRC8 column (Perkin Elmer Inc., Norwalk, CT, USA) with a stepwise gradient at a flow rate of 4 mL/min. The gradient is as follows:
0 - 0.5 minutes, 70% A / 30% B;
0.5 - 3.0 minutes, 67% A / 33% B;
3.0 - 5.0 minutes, 20% A / 80% B;
5.0 - 6.5 minutes, 70% A / 30% B;
where A is 3 mM sodium dodecyl sulfate / 0.05% H$_3$PO$_4$ in H$_2$O and B is 0.05% H$_3$PO$_4$ in acetonitrile. Elution was monitored at 210 nm. (5) A control, which was the assay mixture without the test compound, was subjected simultaneously to steps 2 to 4.

Inhibition Studies:

Cleavage products and remaining parent substrate were quantified by either peak height or by integration of the appropriate HPLC peaks. Substrate conversion was calculated using the following relationship:

$$\% \text{ Conversion} = \frac{\text{Sum of peak height or peak area of products}}{\text{Sum of peak height or peak area of substrate and products}} \times 100$$

Enzyme inhibition of the test compound was calculated as follows:

$$\% \text{ Inhibition} = 100 - \frac{\% \text{ Conversion for assay mixture}}{\% \text{ Conversion of control}} \times 100$$

The concentration of the test compound which causes a 50% inhibition of the HIV-protease, i.e. the IC$_{50}$, was determined as follows: The percent inhibition of the enzyme was determined for a minimum of three different concentrations of the test compound. Thereafter, the IC$_{50}$ was determined graphically by plotting the percent inhibition of the enzyme against the concentration of the test compound.

The following table of exemplified compounds of formula 1 lists their IC$_{50}$ as determined in the recombinant HIV protease HPLC assay.

| Compound | Example in which compound is prepared | IC$_{50}$ (nM) |
|---|---|---|
| Boc-Pheψ[CH$_2$NH]Leu-Glu-NHCH$_2$CH$_2$CH$_2$Ph | 1 | 36 |
| Boc-Pheψ[CH$_2$NH]Cpa-Glu-NHCH$_2$CH$_2$CH$_2$Ph | 2 | 13 |
| Boc-ACHPA-Glu-NHCH$_2$CH$_2$CH$_2$Ph | 4 | 7 |
| Boc-ACHPA-Glu(OMe)-NHCH$_2$CH$_2$CH$_2$Ph | 5 | 110 |
| (C$_2$H$_5$)$_2$CHC(O)-ACHPA-Glu-NHCH$_2$CH$_2$Ph | 6 | 93 |
| (Cyclohexylcarbonyl)-ACHPA-Glu-NHCH$_2$CH$_2$Ph | 12 | 130 |
| Boc-ACHPA-Glu-NH(CH$_2$)$_7$CH$_3$ | 13 | 40 |
| Boc-ACPPA-Glu-NHCH$_2$CH$_2$CH$_2$Ph | 18 | 180 |
| Boc-ACHPA-Glu-NHCH$_2$CH$_2$-(4MeS-Ph) | 22 | 18 |
| Boc-ACHPA-Glu-OCH$_2$CH$_2$CH$_2$Ph | 25 | 85 |
| (CH$_3$)$_3$CNHC(O)-ACHPA-Glu-NHCH$_2$CH$_2$CH$_2$Ph | 26 | 120 |
| Boc-ACHPA-Gluψ[CSNH]-NHCH$_2$CH$_2$CH$_2$Ph | 31 | 380 |

Example 35

Other examples of peptides within the scope of this invention are listed hereinafter together with their IC$_{50}$ (shown in parenthesis) as determined in the recombinant HIV protease HPLC assay:

Boc-ACHPA-Glu-NHCH$_2$CH$_2$CH$_2$-(4F-Ph) (5.6 nM),
Boc-ACHPA-Glu-NHCH$_2$CH$_2$-(4-Cl-Ph) (12 nM),
Moc-ACHPA-Glu-NHCH$_2$CH$_2$CH$_2$Ph (15 nM),
Boc-ACHPA- {(S)-NHCH[CH$_2$CH(CH$_3$)COOH]}C(O)-NHCH$_2$CH$_2$CH$_2$Ph (18 nM),
Boc-ACHPA-Glu-NHCH$_2$CH$_2$CH$_2$-(2-furyl) (25 nM),
Boc-AHDHA-Glu-NHCH$_2$CH$_2$CH$_2$Ph (32 nM),
Boc-ACHPA-Glu-NHCH$_2$CH$_2$-(4-MeO-Ph) (33 nM),
Boc-ACHPA-Glu-NHCH$_2$CH$_2$CH$_2$-(2-thienyl) (33 nM),
Boc-ACHPA-Glu(OMe)-NHCH$_2$CH$_2$CH$_2$-(4F-Ph) (83 nM),
Boc-ACHPA-Glu(OMe)-NHCH$_2$CH$_2$-(4MeS-Ph) (91 nM),
Boc-Pheψ[CH$_2$NH]Cpa-Glu-NHCH$_2$CH$_2$CH$_2$-(4F-Ph) (8 nM),
Boc-Pheψ[CH$_2$NH]Cpa-Glu-NHCH$_2$CH$_2$-(4F-Ph) (8.4 nM),
Boc-Pheψ[CH$_2$NH]Nle-Glu-NHCH$_2$CH$_2$CH$_2$Ph (12 nM),
Boc(4F-Phe)ψ[CH$_2$NH]Cpa-Glu-NHCH$_2$CH$_2$-(4F-Ph) (15 nM),
Boc-Pheψ[CH$_2$NH]Cpa-Glu-NHCH$_2$CH$_2$Ph (16 nM)
Boc-Pheψ[CH$_2$NH]Val-Glu-NHCH$_2$CH$_{22}$CH$_2$Ph (30 nM)

18

Ipoc-ACHPA-Glu-NH-CH$_2$CH$_2$CH$_2$Ph (6.3 nM)
Boc-ACHPA-Glu-NHCH$_2$CH$_2$O-(2Cl-Ph) (6.6 nM)
Boc-ACHPA-Glu-NHCH$_2$CH$_2$OBzl (7.1 nM)
Boc-HACHPA-Glu-NHCH$_2$CH$_2$CH$_2$Ph (7.7 nM)
Boc-ACHPA-Glu-NHCH$_2$CH$_2$OPh (8.6 nM)
Boc-ACHPA-{(S)-NHCH[CH$_2$C(CH$_3$)$_2$COOH]}C(O)-NHCH$_2$CH$_2$CH$_2$Ph (9.4 nM)
Boc-ChaΨ[CH(OH)CH$_2$]Gly-Glu-NHCH$_2$CH$_2$CH$_2$Ph (14 nM)
Boc-homoACHPA-Glu-NHCH$_2$CH$_2$CH$_2$Ph (68 nM)
Boc-ACHPA-{(S)-NHCH[CH$_2$C(CH$_3$)$_2$COOH]}C(O)-NHCH$_2$CH$_2$CH$_2$Ph (69 nM)
Boc-HACHPA-Glu(OMe)-NHCH$_2$CH$_2$CH$_2$Ph (120 nM)
Boc-ACHPA-Glu(OMe)-NHCH$_2$CH$_2$O-(2Cl-Ph) (140 nM)
Ipoc-ACHPA-Glu(OMe)-NHCH$_2$CH$_2$OBzl (150 nM)
CH$_3$C(O)-ACHPA-Glu-NHCH$_2$CH$_2$CH$_2$Ph (160 nM)
Boc-ACHPA-Glu(OMe)-NHCH$_2$CH$_2$OBzl (260 nM) and
Boc-ChaΨ[CH(OH)CH$_2$]Gly-Glu(OMe)-NHCH$_2$CH$_2$CH$_2$Ph (600 nM)

Example 36

The following protocol, used for screening antiviral effects of the compounds of formula 1 , is adapted from a plaque assay utilizing HTLV-I transformed cells which was developed by Harada et al., supra. HTLV-I transformed cells are used in these assays because of the rapidity with which HIV will replicate in these cells.

1. The test compound is dissolved in dimethylsulfoxide to a concentration of 5 mg/mL. The resultant solution can be stored at 4° until used.

2. The resultant solution is diluted in RPMI 1640 (Gibco Laboratories, St. Lawrence, MA, USA) to four times (4x) the final concentration which is to be tested. Once diluted in RPMI 1640, the solution is used in the cell culture assay within 4 h.

3. The 4x solution (50$\mu$L) is added to triplicate wells of a 96 well flat bottomed microtiter plate. RPMI 50$\mu$L also is added to control wells.

4. C8166 cells (5 x 10$^{-4}$) in 50$\mu$L of HEPES-buffered RPMI 1640, 10% beat inactivated fetal calf serum (FCS), 12.5$\mu$L/ml gentamicin (complete media) are added to all wells.

5. Fifty times TCID$_{50}$ of H9/HTLV-IIIB stock (stored in liquid nitrogen as cell culture supernatant in 50% FCS) in 100$\mu$L of complete media is added to all wells. Infectious titer of virus stocks are as previously determined by end point dilution on C8166 cells. Titer of stocks are stable for 6-12 months when stored at -193° C.

6. Microtiter plates are then placed on level shelves of a 37°, 5% CO$_2$ humidified incubator for 72 h.

7. Plates are then removed and centers of syncytia are counted in each well by low power phase contrast light microscopy. Each cluster of cells which shows evidence of any syncytia formation is counted as one center of syncytia. Control wells should have between 25 and 75 centers of syncytia per well.

8. Percent inhibition of syncytia formation is calculated by the formula:

$$\% \text{ inhibition} = 100 \times \frac{\left(\begin{array}{c}\text{\# syncytial centers} \\ \text{in control wells}\end{array}\right) - \left(\begin{array}{c}\text{\# syncytial centers in} \\ \text{test wells}\end{array}\right)}{\begin{array}{c}\text{\# syncytial center in} \\ \text{control wells}\end{array}}$$

When tested according to this procedure, Boc-PheΨ[CH$_2$NH]Cpa-Glu-NHCH$_2$CH$_2$CH$_2$Ph of example 2 and Boc-ACHPA-Glu-NHCH$_2$CH$_2$CH$_2$Ph of example 4 showed 54% and 96% inhibition at 20$\mu$g/mL.

**Claims**

1. A compound of formula 1

19

X-A-B-Y    (1)

wherein X is $R^1OC(O)$, $R^1NHC(O)$ or $R^1C(O)$ wherein $R^1$ is

(i) lower alkyl,

(ii) lower cycloalkyl,

(iii) (lower cycloalkyl)-(lower alkyl),

(iv) phenyl or phenyl monosubstituted with hydroxy, lower alkyl, lower alkoxy or halo, or

(v) phenyl(lower)alkyl or phenyl(lower)alkyl monosubstituted on the aromatic portion thereof with hydroxy, lower alkyl, lower alkoxy or halo;

A is a derived amino acid radical of the formula $NHCH(R^2)$-T wherein $R^2$ has the same meaning as defined hereinabove for $R^1$ and T is $CH(OH)CH_2C(O)$, $CH(OH)CH_2CH_2C(O)$ or $CH_2NHCH(R^3)C(O)$ wherein $R^3$ has the same meaning as defined hereinabove for $R^1$, or A is a derived amino acid radical of the formula $NHCH[CH(OH)-R^{3A}]CH(OH)CH_2(O)$ wherein $R^{3A}$ has the same meaning as defined hereinabove for $R^1$, the three asymmetric centers of the tertiary carbon atoms depicted for the last said radical having in order the (S), (R) and (S) chiral configurations;

B is $NHCH(R^4)C(U)$ wherein $R^4$ is $CR^5(R^6)CR^7(R^8)C(O)V$ wherein $R^5$, $R^6$, $R^7$ and $R^8$ each independently is hydrogen or lower alkyl and V is hydroxy or lower alykoxy, and U is oxo or thioxo; and

Y is $OR^9$ wherein $R^9$ is

(i) (1-10C)alkyl,

(ii) lower cycloalkyl,

(iii) (lower cycloalkyl)-(lower alkyl),

(iv) phenyl(lower)alkyl or phenyl(lower)alkyl wherein the aromatic portion thereof is monosubstituted with hydroxy, halo, lower alkoxy, lower alkyl, $SO_2NH_2$ or $S(O)_nR^{10}$ wherein n is 0, 1 or 2 and $R^{10}$ is lower alkyl,

(v) Het-lower alkyl wherein Het represents a five or six membered heterocyclic radical containing one to two heteroatoms selected from nitrogen, oxygen and sulfur, or

(vi) $CH_2CN_2O$-$(CH_2)_m$-Ph wherein m is 0 or 1, or $CH_2CH_2O$-$(CH_2)_m$-Ph wherein the aromatic portion thereof is substituted with hydroxy, halo, lower alkoxy or lower alkyl and m is 0 or 1; or

Y is $NR^{11}R^{12}$ wherein $R^{11}$ is hydrogen or lower alkyl and $R^{12}$ has the same meaning as defined hereinabove for $R^9$;

or a therapeutically acceptable salt thereof.

2. A compound as recited in claim 1 wherein X is $R^1OC(O)$, $R^1NHC(O)$ or $R^1C(O)$ wherein $R^1$ is

(i) lower alkyl,

(ii) cyclopropyl, cyclopentyl or cyclohexyl,

(iii) cyclopropylmethyl, cyclohexylmethyl or 2-cyclohexylethyl,

(iv) phenyl, 4-chlorophenyl, 4-methylphenyl or methoxy-phenyl, or

(v) benzyl, (4-chlorophenyl)methyl, (4-methylphenyl)methyl or (4-methoxyphenyl)methyl;

A is $NHCH(R^2)$-T wherein $R^2$ lower alkyl, (lower cycloalkyl)methyl, 2-(lower cycloalkyl)ethyl, benzyl, (4-hydroxyphenyl)methyl, (4-fluorophenyl)methyl, or (4-methoxyphenyl)methyl and T is $CH(OH)CH_2C(O)$, $CH(OH)CH_2CH_2C(O)$ or $CH_2NHCH(R^3)C(O)$ wherein $R^3$ is lower alkyl, cyclopropylmethyl, cyclohexylmethyl, phenyl or benzyl, or A is (S,R,S)-$NHCH[CH(OH)-(lower cycloalkyl)]CH(OH)CH_2C(O)$; B is $NHCH(R^4)C(U)$ wherein $R^4$ is $CH_2CH_2C(O)OH$, $CH_2CH(CH_3)C(O)OH$, $CH(C_2H_5)CH_2C(O)OH$, $CH_2CH_2C(O)OCH_3$, $CH_2CH_2C(O)OC_2H_5$, $CH_2CH(CH_3)C(O)OCH_3$ or $CH(CH_3)CH(CH_3)C(O)OCH_3$ and U is oxo or thioxo; and

Y is $OR^9$ wherein $R^9$ is

(i) (1-10C)alkyl,

(ii) cyclopropyl, cyclopentyl or cyclohexyl,

(iii) cyclopropylmethyl, 2-cyclopropylethyl, 3-cyclopropylpropyl, cyclohexylmethyl, 2-cyclohexylethyl or 3-cyclohexylpropyl,

(iv) phenyl(lower)alkyl or phenyl(lower)alkyl substituted at position 4 of the phenyl with hydroxy, fluoro, chloro, bromo, lower alkoxy, lower alkyl, $SO_2NH_2$, lower alkylthio or lower alkylsulfonyl,

(v) Het-lower alkyl wherein Het is a heterocyclic radical selected from 2-pyrrolyl, 2-furyl, 2-thienyl, 2-isoxazolyl and 2-thiazolyl, or

(vi) $CH_2CH_2O$-$(CH_2)_m$-Ph wherein m is 0 or 1 or $CH_2CH_2O$-$(CH_2)_m$-Ph wherein the aromatic portion thereof is monosubstituted with hydroxy, fluoro, chloro, bromo, lower alkoxy or lower alkyl and m is 0 or 1; or

Y is $NR^{11}R^{12}$ wherein $R^{11}$ is hydrogen or methyl and $R^{12}$ has the same meaning as defined in the last instance for $R^9$; or a therapeutically acceptable salt thereof.

3. A compound as recited in claim 2 wherein X is $R^1OC(O)$ or $R^1C(O)$ wherein $R^1$ is methyl, ethyl, 1-methylethyl, 1,1-dimethylethyl, propyl, 1-methylpropyl, 1-ethylpropyl, 2-methylpropyl, 1,1-dimethylpropyl, 1,2-dimethylpropyl, 2,2-dimethylpropyl, butyl, cyclopropyl, cyclohexyl, cyclopropylmethyl, cyclohexylmethyl, phenyl, 4-methoxyphenyl, benzyl, (4-chlorophenyl)methyl or (4-methoxyphenyl)-methyl; A is $NHCH(R^2)$-T wherein $R^2$ is 1-methylethyl, propyl, 1-methylpropyl, 2-methylpropyl, 1,1-dimethylethyl, 2,2-dimethylpropyl, butyl, cyclopropylmethyl, cyclohexylmethyl, 2-cyclohexylethyl, benzyl, (4-hydroxyphenyl)methyl, (4-fluorophenyl)methyl or (4-methoxyphenyl)methyl, and T is $CH(OH)CH_2C(O)$, $CH(OH)CH_2CH_2C(O)$ or $CH_2NHCH(R^3)C(O)$ wherein $R^3$ is 1-methylethyl, propyl, 1-methylpropyl, 2-methylpropyl, 1,1-dimethylethyl, butyl, cyclopropylmethyl, cyclohexylmethyl, phenyl or benzyl, or A is $(S,R,S)$-$NHCH[CH(OH)cyclohexyl]CH(OH)CH_2C(O)$; B is $NHCH(R^4)C(U)$ wherein $R^4$ is $CH_2CH_2C(O)OH$, $CH_2CH_2C(O)OMe$, $CH_2CH(CH_3)C(O)OH$ or $CH_2CH$-$(CH_3)C(O)OCH_3$ and U is oxo; and Y is methoxy, ethoxy, hexyloxy, octyloxy, cyclopropyloxy, 3-cyclopropylpropoxy, 2-cyclohexylethoxy, 3-cyclohexylpropoxy, 2-phenylethoxy, 2-[4-(aminosulfonyl)-phenyl]ethoxy, 2-(4-fluorophenyl)ethoxy, 2-[4-(methylthio)phenyl]ethoxy, 3-phenylpropoxy, 3-(4-fluorophenyl)propoxy, 3-(4-chlorophenyl)propoxy, 3-(4-hydroxyphenyl)propoxy, 3-(4-propoxyphenyl)-propoxy, 3-[4-(methylthio)phenyl]propoxy, 3-[4-(methylsulfonyl)phenyl]propoxy,2-(phenoxy)ethoxy, 2-(2-chlorophenoxy)ethoxy, 2-(4-methylphenoxy)ethoxy, 2-phenylmethoxy)ethoxy, butylamino, hexylamino, octylamino, cyclopropylamino, 3-cyclopropylpropylamino, 2-cyclohexylethylamino, 3-cyclohexylpropylamino, 2-phenylethylamino, 2-[4-(aminosulfonyl)-phenyl]ethylamino, 2-(4-fluorophenyl)ethylamino, 2-(4-chlorophenyl)ethylamino, 2-[4-(methylthio)phenyl]ethylamino, 2-(4-methoxyphenyl)ethylamino, 3-phenylpropylamino, 3-(4-fluorophenyl)propylamino, 3-(4-chlorophenyl)propylamino, 3- (4-hydroxyphenyl)propylamino, 3-(4-methoxyphenyl)propylamino, 3-(4-propoxyphenyl)propylamino, 3-[4-(methylthio)phenyl]propylamino, 3-[4-(methylsulfonyl)phenyl]propylamino, 3-(2-furyl)propylamino, 3-(2-thienyl)propylamino, 2-(phenoxy)ethylamino, 2-(2-chlorophenoxy)ethylamino, 2-(4-methylphenoxy)-ethylamino or 2-(phenylmethoxy)ethylamino; or a therapeutically acceptable salt thereof.

4. A compound as recited in claim 3 wherein X is methoxycarbonyl, 1-methylethoxycarbonyl, 1,1-dimethylethoxycarbonyl, 1-oxobutyl, 3-methyl-1-oxobutyl, 2-ethyl-1-oxobutyl, 2,2-dimethyl-1-oxobutyl, 2,3-dimethyl-1-oxobutyl, 3,3-dimethyl-1-oxobutyl or cyclohexylcarbonyl; A is the radical $NHCH(R^2)CH$-$(OH)CH_2C(O)$ wherein $R^2$ is 2,2-dimethylpropyl, butyl, cyclopropylmethyl, cyclohexylmethyl or 2-cyclohexylethyl, the two asymmetric centers of the last said radical having the (S) chiral configurations, or A is the radical $(NHCH(R^2)CH_2NHCH(R^3)C(O)$ wherein $R^2$ is cyclohexylmethyl, benzyl or (4-fluorophenyl)methyl and $R^3$ is 1-methylethyl, 2-methylpropyl, butyl or cyclopropylmethyl, the two asymmetric centers of the last said radical having the (S) chiral configuration, or A is $(S,R,S)$-$NHCH$-$[CH(OH)cyclohexyl]CH(OH)CH_2C(O)$; B is Glu, Glu(OMe) or $NHCH[CH_2CH(CH_3)COOH]C(O)$;and Y is 2-phenylethoxy, 3-phenylpropoxy, hexylamino, octylamino, 3-cyclohexylpropylamino, 2-phenylethylamino, 2-(4-fluorophenyl)ethylamino, 2-(4-chlorophenyl)ethylamino, 2-[4-(methylthio)phenyl]ethylamino, 2(4-methoxyphenyl)ethylamino, 3-phenylpropylamino, 3(4-fluorophenyl)propylamino, 3-(4-hydroxyphenyl)-propylamino, 3-(4-propoxyphenyl)propylamino, 3-[4-(methylthio)phenyl]propylamino, 3(2-furyl)-propylamino, 3-(2-thienyl)propylamino, 2-(phenoxy)ethylamino, 2-(2-chloro-phenoxy)ethylamino or 2-(phenylmethoxy)ethylamino; or a therapeutically acceptable salt thereof.

5. A compound as recited in claim 1 selected from the group of:

Boc-PheΨ[$CH_2NH$]Leu-Glu-$NHCH_2CH_2CH_2Ph$
Boc-PheΨ[$CH_2NH$]Cpa-Glu-$NHCH_2CH_2CH_2Ph$
$(C_2H_5)_2CHC(O)$-PheΨ[$CH_2NH$]Cpa-Glu-$NHCH_2CH_2CH_2Ph$
Boc-ACHPA-Glu-$NHCH_2CH_2CH_2Ph$
Boc-ACHPA-Glu-(OMe)-$NHCH_2CH_2CH_2Ph$
$(C_2H_5)_2CHC(O)$-ACHPA-Glu-$NHCH_2CH_2Ph$
Boc-ACHPA-Glu-$NHCH_2CH_2Ph$
$CH_3CH_2CH_2C(O)$-ACHPA-Glu-$NHCH_2CH_2Ph$
$CH_3CH(CH_3)CH_2C(O)$-ACHPA-Glu-$NHCH_2CH_2Ph$
$(Cyclohexyl)C(O)$-ACHPA-Glu-$NHCH_2CH_2Ph$

Boc-ACHPA-Glu-NH(CH$_2$)$_7$CH$_3$
Boc-NorSta-Glu-NHCH$_2$CH$_2$CH$_2$Ph
Boc-ACHPA-Glu-NHCH$_2$CH$_2$CH$_2$-(4HO-Ph)
Boc-ACHPA-Glu-NHCH$_2$CH$_2$-(4F-Ph)
Boc-ACHPA-Glu-NHCH$_2$CH$_2$-(4-MeS-Ph)
Boc-ACHPA-Glu-NHCH$_2$CH$_2$CH$_2$-(4-PrO-Ph)
Boc-ACHPA-Glu-OCH$_2$CH$_2$CH$_2$Ph
Boc-ACHPA-Glu-NHCH$_2$CH$_2$CH$_2$-(4F-Ph)
Moc-ACHPA-Glu(OMe)-NHCH$_2$CH$_2$CH$_2$Ph
Boc-ACHPA-Glu-NHCH$_2$CH$_2$-(4Cl-Ph)
Moc-ACHPA-Glu-NHCH$_2$CH$_2$CH$_2$Ph
Boc-ACHPA-{(S)-NHCH[CH$_2$CH(CH$_3$)COOH]} C(O)-NHCH$_2$CH$_2$CH$_2$Ph
Boc-ACHPA-Glu-NHCH$_2$CH$_2$CH$_2$-(2-furyl)
Boc-AHDHA-Glu-NHCH$_2$CH$_2$CH$_2$Ph
Boc-ACHPA-Glu-NHCH$_2$CH$_2$(4-MeO-Ph)
Boc-ACHPA-Glu-NHCH$_2$CH$_2$CH$_2$(2-thienyl)
Boc-ACHPA-Glu(OMe)-NHCH$_2$CH$_2$CH$_2$-(4F-Ph)
Boc-ACHPA-Glu(OMe)-NHCH$_2$CH$_2$-(4MeS-Ph)
Boc-PheΨ[CH$_2$NH]Cpa-Glu-NHCH$_2$CH$_2$CH$_2$-(4F-Ph)
Boc-PheΨ[CH$_2$NH]Cpa-Glu-NHCH$_2$CH$_2$-(4F-Ph)
Boc-PheΨ[CH$_2$NH]Nle-Glu-NHCH$_2$CH$_2$CH$_2$Ph
Boc-(4F-Phe)Ψ[CH$_2$NH]Cpa-Glu-NHCH$_2$CH$_2$-(4F-Ph)
Boc-PheΨ[CH$_2$NH]Cpa-Glu-NHCH$_2$CH$_2$Ph
Boc-PheΨ[CH$_2$NH]Val-Glu-NHCH$_2$CH$_2$CH$_2$Ph
Ipoc-ACHPA-Glu-NH-CH$_2$CH$_2$CH$_2$Ph
Boc-ACHPA-Glu-NHCH$_2$CH$_2$O-(2Cl-Ph)
Boc-ACHPA-Glu-NHCH$_2$CH$_2$OBzl
Boc-HACHPA-Glu-NHCH$_2$CH$_2$CH$_2$Ph
Boc-ACHPA-Glu-NHCH$_2$CH$_2$OPh
Boc-ACHPA-{(S)-NHCH[CH$_2$C(CH$_3$)$_2$COOH]}C(O)NHCH$_2$CH$_2$CH$_2$Ph
Boc-ChaΨ[CH(OH)CH$_2$]Gly-Glu-NHCH$_2$CH$_2$CH$_2$Ph
Boc-homoACHPA-Glu-NHCH$_2$CH$_2$CH$_2$Ph
Boc-ACHPA-{(S)-NHCH[CH$_2$C(CH$_3$)$_2$COOH]}C(O)-NHCH$_2$CH$_2$CH$_2$Ph
Boc-HACHPA-Glu(OMe)-NHCH$_2$CH$_2$CH$_2$Ph
Boc-ACHPA-Glu(OMe)-NHCH$_2$CH$_2$O-(2Cl-Ph)
Ipoc-ACHPA-Glu(OMe)-NHCH$_2$CH$_2$OBzl
CH$_3$C(O)-ACHPA-Glu-NHCH$_2$CH$_2$CH$_2$Ph
Boc-ACHPA-Glu(OMe)-NHCH$_2$CH$_2$OBzl and
Boc-ChaΨ[CH(OH)CH$_2$]Gly-Glu(OMe)-NHCH$_2$CH$_2$CH$_2$Ph

6.  A pharmaceutical composition comprising a compound as recited in claim 1, or a therapeutically acceptable salt thereof, and a pharmaceutically acceptable carrier.

7.  The use of a compound as recited in anyone of claims 1 to 5, or a therapeutically acceptable salt thereof for the manufacture of a medicament for treating HIV infections in a human.

8.  The use of a compound as recited in anyone of claims 1 to 5, or a therapeutically acceptable salt thereof for the manufacture of a medicament for protecting human cells against HIV pathogenesis.

9.  A process for preparing a compound of formula 1 as recited in claim 1 comprising:
    coupling a first fragment of formula X-A-OH wherein X and A are as defined in claim 1 with a second fragment of formula H-B$^1$-Y wherein B$^1$ is the radical NHCH(R$^{4A}$)C(U) wherein R$^{4A}$ is CR$^5$(R$^6$)CR$^7$(R$^8$)C-(O)V$^1$ wherein R$^5$ to R$^8$, inclusive, are independently hydrogen or lower alkyl and V$^1$ is a carboxy protective group and U is oxo or thioxo, and Y is as defined in claim 1 to obtain the corresponding protected intermediate of formula X-A-B$^1$-Y wherein X, A, B$^1$ and Y are as defined herein: followed by subjecting the protected intermediate to deprotection, and if required to resolution or to esterification and resolution, to obtain the corresponding compound of formula 1; and, if desired, transforming the compound into a therapeutically acceptable salt.

22